# EUROPEAN PATENT APPLICATION

(11) **EP 2 522 368 A1**
(43) Date of publication of application: **14.11.2012**
(21) Application number: 11382147.4
(22) Date of filing: 13.05.2011
(51) Int. Cl.: A61K 49/00

(54) **Microparticles for intraocular tissue marking**

(71) Applicant: Bioftalmik, S.L., 48160 Derio (Vizcaya) (ES)
(72) Inventor: Vega Chapparo, Sandra Clarissa, E-48160 Derio - Vizcaya (ES); Suárez Cortés, Tatiana, E-48160 Derio - Vizcaya (ES); Corcóstegui Crespo, Iñigo, E-48006 Bilbao - Vizcaya (ES)
(74) Representative: ABG Patentes, S.L.

(57) **Abstract**

The invention describes a composition comprising a plurality of microparticles of a biodegradable polymer with size and surface porosity characteristics useful for viewing intraocular tissues and which can be applied in ocular surgery.

## Description

### Field of the Invention

The invention is comprised in the field of ocular surgery particularly in vitreoretinal surgical procedures. More specifically, the invention relates to ophthalmic compositions useful as contrast media for viewing the vitreous body of an eye during a vitrectomy and to methods for using said ophthalmic compositions.

### Background of the Invention

The vitreous is a clear, transparent, semisolid gel taking up two-thirds of the volume of the eyeball extending from the posterior face of the lens to the inner surface of the retina and surrounded by the hyaloid membrane. The vitreous humor is formed in the normal human eye by connective tissue with approximately 99% of water and the remaining 1% is made up of intercellular collagen molecules, hyaluronic acid, soluble glycoproteins and other low molecular weight metabolites. Vitrectomy is the surgical extraction of the vitreous, and it is one of the most frequently used therapeutic procedures in ophthalmology. Vitrectomy is a complex surgical technique performed to treat pathologies such as retinal detachment or macular hole. The treatment of said diseases depends on the effectiveness and safety with which the vitreous is extracted; therefore, the clear delineation of particular retinal structures and of the vitreous during vitrectomy helps the surgeon and improves the results of the surgical procedure.

The application of this surgical technique (vitrectomy) is sometimes difficult due to the lack of contrast that the intraocular tissues may present. To overcome this, different types of stains have been developed to attempt to improve the viewing and contrast of the intraocular structures and to thereby facilitate surgery. The term "chromovitrectomy" thus arises, which is a concept that refers to the use of substances which facilitate viewing intraocular structures during a vitrectomy (surgery of the fundus of the eye); said substances include the so-called vital dyes". Vital dyes are those which are capable of modifying the color of living tissue or collagen fibers causing minimal damage to the living organism. The so-called "vital dyes" include indocyanine green, fluorescein sodium, trypan blue, infracyanine green, brilliant blue, patent blue and corticoids, such as triamcinolone acetonide which, though they are used as dyes, have generated controversy with respect to safety and toxicity [Gale, J.S., et al., Comparison of the in vitro toxicity of indocyanine green to that of trypan blue in human retinal pigment epithelium cell cultures. Am J Ophthalmol, 2004. 138(1):64-9; Haritoglou, C., et al., Indocyanine green-assisted peeling of the internal limiting membrane in macular hole surgery affects visual outcome: a clinicopathologic correlation. Am J Ophthalmol, 2002. 134(6):836-41; Veckeneer, M., et al., Ocular toxicity study of trypan blue injected into the vitreous cavity of rabbit eyes. Graefes Arch Clin Exp Ophthalmol, 2001. 239(9):698-704].

An alternative for preventing the drawbacks associated with the use of said dyes comprises the use of particular systems. In that respect the use of polylactic acid (PLA) granules with a mean diameter of approximately 20 µm as a surgical adjuvant for viewing the vitreous body during vitrectomy has been described because said PLA granules adhere to the surface of the vitreous, leading to it being seen as a white membrane [Yamashita, T et al. Invest Ophthalmol Vis Sci. 2007; 48(7):3277-82].

The use of lactic acid-glycolic acid copolymer or poly-(lactic-co-glycolic) acid (PLGA) microparticles for viewing the vitreous body has also been described:
- European patent application EP 1752152 discloses preparations and methods for viewing transparent tissues of the eye (including the vitreous body) during a surgical operation. Said preparations comprise particles of a macromolecular compound, for example PLGA. The document describes obtaining PLGA microparticles with a mean size of 15.5 µm which are suspended in an intraocular irrigation solution to produce a preparation from which larger-sized particles are removed by means of centrifugation and to form a supernatant containing PLGA microparticles with a mean size of 3.1 µm. Other PLGA preparations the mean particle size of which is 0.06-0.07 µm are further described; and
- United States patent application US 2007/0218007 discloses compositions comprising a plurality of particles of a polymeric material, for example PLGA, with a mean particle size of approximately 10-20 µm of diameter as a contrast component.

None of said documents describes the effect of the distribution of the size of said microparticles or their outer surface characteristics on viewing transparent intraocular structures. For this reason, these latter PLGA-based microparticulate systems do not induce a distribution effect in the vitreous based on the aforementioned physicochemical properties relating to the composition of the vitreous body to thereby allow precise viewing of intraocular structures, particularly of the vitreous body or of vitreoretinal structures during a vitrectomy.

Therefore, there continues to be a need to develop new systems useful as contrast media for use in ocular surgery, particularly in vitrectomy, which allow increasing the contrast of vitreoretinal structures without causing harmful or toxic effects for the purpose of reducing the surgical risk and increasing the effectiveness of the vitrectomy. Said system should advantageously be opaque or semi-opaque, must be injectable into the vitreous cavity with the surgical material used in routine surgeries of this type, must be able to act as a delineating agent of vitreoretinal structures, and must be biocompatible and biodegradable.

### Summary of the Invention

It has now surprisingly been found that the use of microparticles prepared from a biocompatible and biodegradable polymer with particular size, particle size distribution and outer surface features as a contrast medium in ocular surgery, allows improving viewing transparent or translucent tissues, for example the vitreous body, during a surgical procedure, such as during a vitrectomy, because said microparticles describe a differential distribution within the entire gelatinous mass of the vitreous body which allows increasing the contrast of vitreoretinal structures without producing harmful or toxic effects, and it further allows clearly viewing and delineating vitreoretinal structures, which enables reducing the surgical risk for the patient and increasing the effectiveness of the vitrectomy.

It has particularly been observed that the combination of microparticles prepared from a biodegradable and biocompatible polymer with a mean particle diameter comprised between 10 and 60 µm and a smooth surface (i.e., without pores on its surface) with microparticles prepared from a biodegradable and biocompatible polymer with a mean particle diameter comprised between 10 and 60 µm and a porous surface (i.e., with pores on its surface) in a particular ratio, typically between 10% and 40% of the total microparticles present in said combination are microparticles with a porous surface, it has optimal features for being used as a contrast medium in ocular surgery, particularly for viewing transparent or translucent tissues, for example the vitreous. On one hand, the porous surface structure provided by the microparticles with a porous surface contributes to the dynamics and distribution of the product inside the vitreous and induces hydrophilic properties in said combination and, on the other hand, the compact structure provided by the microparticles with a smooth surface allows delineating the vitreous close to the inner limiting membrane (ILM) as a result of a precipitation or sedimentation phenomenon thereof. The fact that they do not contain any chemical dye or preservative likewise allows the ophthalmic compositions comprising said microparticle combination to be a safer product.

To obtain the microparticle combination with particular features in defined proportions provided by this invention in which each type of microparticles has been chosen to provide and enhance the volume and size characteristics necessary for generating optimal contrast in the vitreous either due to the effect of dispersion through the vitreous or due to precipitation in the retina, various *in vitro* and ex *vivo* assays were conducted in which each of said types of microparticles were individually evaluated and classified (preliminary assays) according to their physicochemical and morphometric characteristics and to their performance in the vitreous of enucleated pig eyes. Based on the results obtained several mixtures of different types of microparticles were designed which were in turn comparatively characterized *ex vivo*, evaluating their performance in the vitreous, using the commercial product currently used as a vital dye in the routine practice of vitrectomies as a positive control, specifically, the triamcinolone (TA). The different assays conducted showed that some mixtures provided highly effective results as a contrast medium during vitrectomy, as illustrated in Examples included in the present description.

The inventive aspects of the present invention are described in the attached claims.

### Brief Description of the Drawings

Figure 1 shows the mean diameter distribution histograms of different batches of PLGA microparticles provided by this invention, determined by means of laser diffraction. The results corresponding to batches V005-B2 (Figure 1A), V009-43 (Figure 1B) and V016-40 (Figure 1C) and of Mixture 5 (Figure 1D) formed by the combination of specific amounts of said batches of microparticles, are shown.
Figure 2 is a graph showing the mean particle diameter distribution analysis by means of laser diffraction of the different batches of microparticles forming Mixture 5 [V005-B2, V009-43 and V016-40] in addition to Mixture 5, together.
Figure 3 consists of a set of photographs showing the morphological characterization of the microparticles forming Mixture 5 [V005-B2 (Figure 3A), V009-43 (Figure 3B) and V016-40 (Figure 3C)] and in addition to Mixture 5 (Figure 3D) by means of scanning electron microscopy (SEM) showing the different sizes of the populations of microparticles in each sample.
Figure 4 consists of a set of photographs showing the morphological characterization of the microparticles forming Mixture 5 [V005-B2 (Figure 4A), V009-43 (Figure 4B) and V016-40 (Figure 4C)] in addition to Mixture 5 (Figure 4D) by means of SEM in which the morphology of the microparticles present in each batch is described.
Figure 5 consists of a set of photographs showing the morphological characterization of the microparticles forming Mixture 5 [V005-B2 (Figure 5A), V009-43 (Figure 5B) and V016-40 (Figure 5C)] in addition to Mixture 5 (Figure 5D) by means of SEM in which the surface of the microparticle is detailed and in some cases the inside of the microparticles is denoted. Figure 5D shows a 100X magnification to easily observe the different populations of microparticles with respect to size and outer surface.
Figure 6 consists of a set of photographs showing the degradation kinetics of a batch of PLGA microparticles (V005-B2) [aqueous phase in the production of the W/O emulsion: physiological solution], followed by means of SEM, at days: 0 (Figure 6A), 7 (Figure 6B), 14 (Figure 6C) and 21 (Figure 6D).
Figure 7 is a graph showing the effect of a dose of PLGA microparticles on ARPE-19 cell proliferation.
Figure 8 is a graph showing the effect of repeated doses of PLGA microparticles on the ARPE-19 cell proliferation.
Figure 9 is a set of microphotographs illustrating the application of TA during vitrectomy in enucleated pig eyes.
Figure 10 is a set of microphotographs illustrating the application of a composition of PLGA microparticles representative of the present invention (Mixture 5) during vitrectomy in enucleated pig eyes.
Figure 11 is a set of microphotographs in which it can be seen how the PLGA microparticles of a composition of microparticles representative of the present invention (Mixture 5) delineate the retina as a result of precipitation of the microparticles.
Figure 12 is a set of microphotographs in which small vitreous fibers can be seen in the posterior segment of the eye after applying a composition of PLGA microparticles representative of the present invention (Mixture 5).
Figure 13 is a photograph of a rabbit's eye in which a slight conjunctival reaction (conjunctival hyperemia) can be seen during the first week post-injection of a composition of PLGA microparticles representative of the present invention (V007-43).
Figure 14 is a photograph showing the retina under indirect ophthalmoscopy of a rabbit's eye into which a composition of PLGA microparticles representative of the present invention (V007-43) has been injected; no signs of inflammation, bleeding or ischemia are observed.
Figure 15 shows ocular tissue histology results after 1 month post intravitreal injection of a composition of PLGA microparticles representative of the present invention (V007-43); a blackish dust adhered close to the pars plana and in the most peripheral part of the vitreous humor and cell debris in the periphery of the vitreous humor (Figure 15A) can be seen. Figure 15B shows the histology of the retina where no inflammatory reactions or microparticle deposits are observed.

### Detailed Description of the Invention

In one aspect, the invention relates to a composition, hereinafter composition of the invention, comprising a plurality of microparticles of a biodegradable polymer wherein said microparticles have a mean particle diameter comprised between 10 and 60 µm and wherein between 10% and 40% of the total of said microparticles have a porous surface.

As it is used herein, the term "microparticle" includes solid particle systems and suspensions prepared from a polymer, particularly matrix microspheres, with a mean particle diameter comprised between 1 and 999 micrometers (µm), although to put the present invention into practice said microparticles are formed by a biodegradable polymer and have a mean particle diameter comprised between 10 and 60 µm, preferably between 15 and 50 µm, more preferably between 20 and 40 µm.

As it is used herein, the term "mean particle diameter" or "mean particle size" refers to the mean diameter of a microparticle population which is determined in an aqueous suspension. The mean diameter of these systems can be measured by conventional methods known by persons skilled in the art and described by way of illustration in Examples, for example laser diffraction (for a precise determination) or optical microscopy (for a rough but estimated determination). The mean particle diameter can mainly be affected by the amount and the molecular weight of the polymer and by some parameters of its production process.

As it is used herein, the term "polymer" includes both homopolymers and copolymers, i.e., polymers including units of 2, 3, 4 or even more monomers and the like and mixtures thereof. Said polymer must be pharmacologically inactive on ocular tissues in mammals, especially in human beings, and do not cause allergic reactions, immune reactions, etc., in contact with the tissues of the organism, i.e., biocompatible, and it must further be biodegradable, i.e., absorbable by the organism or susceptible to being broken down fairly quickly by the organism into the chemical elements that form it. There is no particular limitation as to the molecular weight of said polymer for use in the present invention, it must simply be solid at room temperature and able to form microparticles. The molecular weight of the polymer is typically equal to or greater than 500, preferably equal to or greater than 1,000, more preferably equal to or greater than 1,500, even more preferably equal to or greater than 2,000. Likewise, the molecular weight of the polymer is typically equal to or less than 200,000, preferably equal to or less than 150,000, more preferably equal to or less than 100,000, even more preferably equal to or less than 50,000. Nevertheless, polymers with molecular weights outside the limits indicated can also be used provided that they can serve to achieve the objective of the present invention. Illustrative non-limiting examples of biodegradable polymers that can be used within the context of the present invention include fatty acid polyesters such as polylactic acid (PLA), polyglycolic acid (PGA), lactic acid-glycolic acid or poly-(lactic-co-glycolic) acid (PLGA) copolymer, polyhydroxybutyric acid, polyhydroxyvaleric acid, polycaprolactone, hydroxybutyric acid-glycolic acid copolymer, lactic acid-caprolactone copolymer, polyethylene succinate, polybutylene succinate, etc.; polysaccharides, such as starch and starch derivatives, including soluble starch, pregelatinized starch and the like, cellulose and cellulose derivatives such as acetylcellulose, ethylcellulose, hydroxypropyl methylcellulose and the like, chitosan, chitin, dextran and the like. In a particular embodiment, said biodegradable polymer is PLGA, a biocompatible material with easy and fast (days) biodegradation (the metabolism and elimination of PLGA occurs by means of non-enzymatic hydrolysis and generates lactic acid and glycolic acid as end products, which are eliminated from the human body naturally by means of the Krebs cycle), which suitably spread throughout the vitreous and provides suitable opacity to delineate the vitreous structures and it has proven to be safe when injected into the eye [Harris, J.M. & R.B. Chess, Effect of pegylation on pharmaceuticals. Nat Rev Drug Discov, 2003. 2(3):214-21; Jain, R.A., The manufacturing techniques of various drug loaded biodegradable poly(lactide-co-glycolide) (PLGA) devices. Biomaterials, 2000. 21(23):2475-90; Middleton, J.C. & A.J. Tipton, Synthetic biodegradable polymers as orthopedic devices. Biomaterials, 2000. 21(23):2335-46].

For the purposes of the invention the composition of the invention will advantageously have a refractive index different from the vitreous in order to create contrast and to facilitate viewing the vitreous; this can be achieved, for example by using water-insoluble polymers, such as PLGA, the aqueous suspensions of which have a refractive index different from the vitreous, which translates into a white contrast or stain in vitreous tissue induced by the PLGA microparticles, thereby providing excellent contrast *in situ.*

In a particular embodiment, all the microparticles present in the composition of the invention are formed by the same biodegradable polymer. In another particular embodiment, the microparticles present in the composition of the invention can be formed by different biodegradable polymers.

The microparticles of the composition of the invention have a mean particle diameter comprised between 10 and 60 µm, typically between 20 and 40 µm. The mean particle diameter can be determined by conventional techniques known by persons skilled in the art, for example by means of laser diffraction.

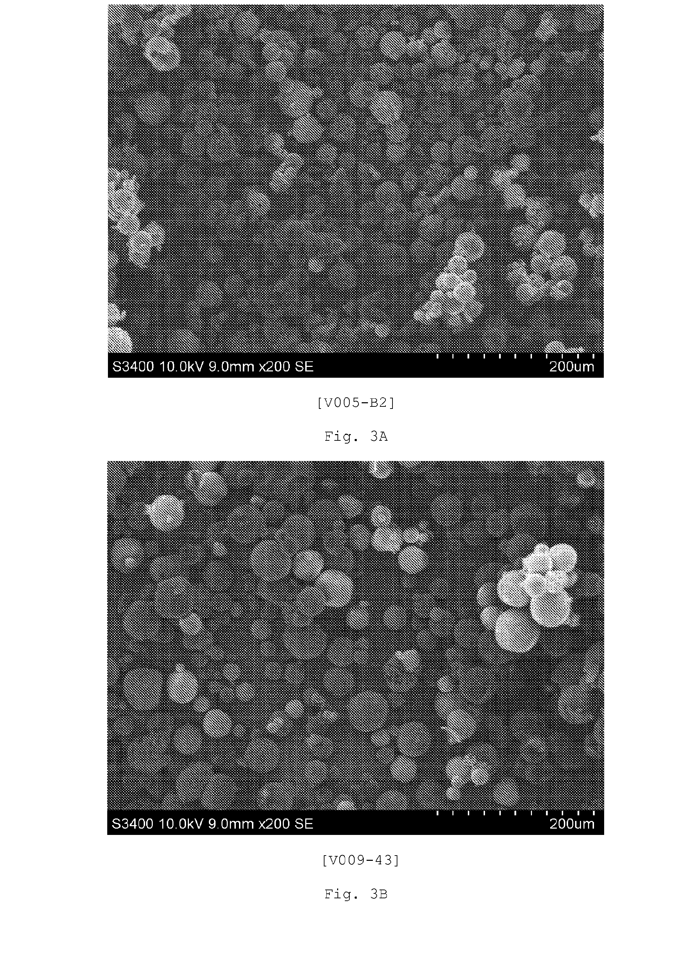

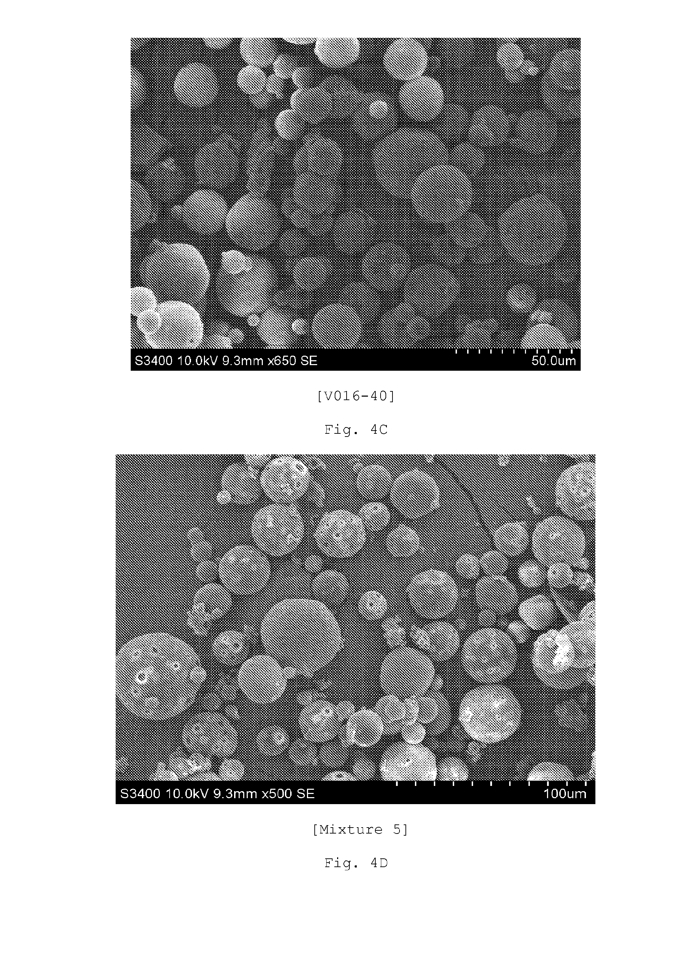

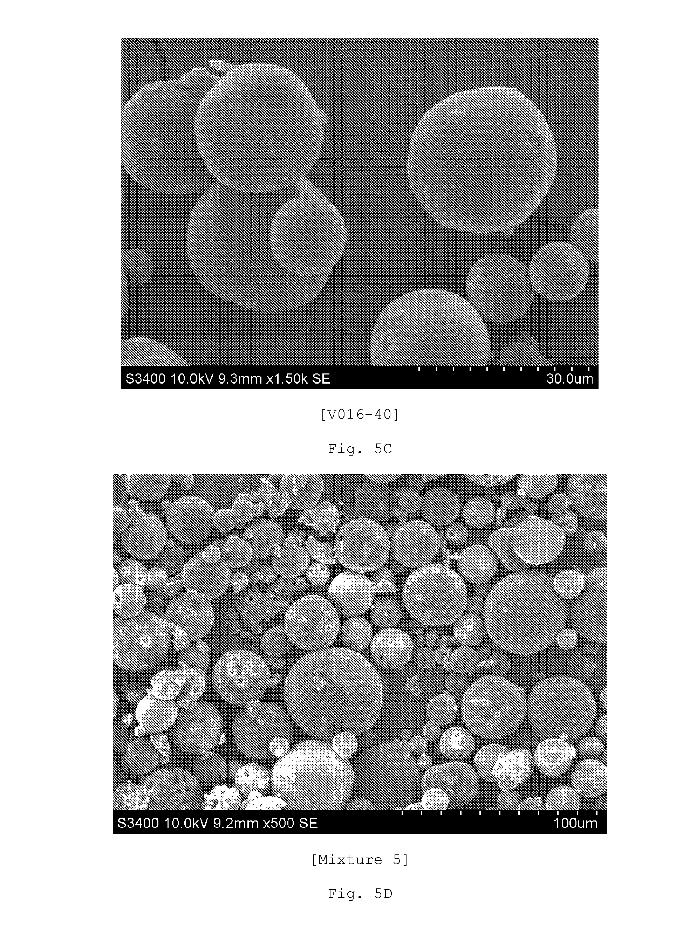

Also according to the present invention, between 10% and 40% of the total biodegradable polymer microparticles with a mean particle diameter comprised between 10 and 60 µm have a porous surface, i.e., they have pores on their surface, as observed, for example in Figures 3A, 4A and 5A, which show the morphological characterization of the batch of microparticles identified as V005-B2, used in the preparation of a composition of the invention identified as Mixture 5, comprising a mixture of microparticles with a porous surface and microparticles with a smooth surface, as seen in Figures 4D and 5D. These microparticles having a porous surface can be obtained by means of a conventional solvent - double emulsion evaporation/extraction process in which the first emulsion (W/O) is formed by mixing the biodegradable polymer solution (or suspension) with an aqueous phase containing electrolytes, for example a 0.9% NaCl aqueous solution, as described below and in Example 1. The percentage of microparticles with a porous surface can be determined by conventional methods; by way of illustration, a homogenous sample of microparticles representative of the composition of microparticles to be analyzed can be subjected to scanning electron microscopy (SEM) and the microparticles containing pores on their surface and the total microparticles in the obtained microphotograph can be counted and the proportion of porous microparticles with respect to the total microparticles can be determined. This determination can be performed manually or by using computer tools, for example by means of the PAX-it! Image Analysis Software program [http://www-paxit.com.paxit/enhanced.asp]. Other methods for determining the porosity of the microparticles have been described by Ehtezazi *et al.* [Ehtezazi C et al., Determination of the internal morphology of poly (D,L-lactide) microspheres using stereological methods. J Control Release. 1999 Feb 22;57(3):301-14].

The composition of the invention comprises a plurality of microparticles of a biodegradable polymer with a mean particle diameter comprised between 10 and 60 µm, of which between 10% and 40% of the total of said microparticles have a porous surface, whereas the rest, between 90% and 60%, respectively, of said microparticles have a smooth surface, i.e., without surface pores. The composition of the invention can be obtained by conventional methods, for example by mixing the corresponding amounts of (i) microparticles with the suitable mean diameter and a smooth surface and (ii) microparticles with the suitable mean diameter and a porous surface.

Both of the microparticles (e.g., microparticles with a smooth surface) as well as the others (e.g., microparticles with a porous surface) can be obtained by conventional methods with slight modifications, as described in Example 1. By way of illustration, said microparticles can be obtained by means of the solvent - double emulsion evaporation/extraction process. Briefly, a solution (or suspension) of the biodegradable polymer in an organic solvent, for example in dichloromethane (DCM), is emulsified with an aqueous phase to form a W/O type emulsion. Assays conducted by the inventors have surprisingly shown that when the aqueous phase is formed from substantially electrolytefree water, for example sterile water for injection, mostly microparticles with a smooth surface are formed; however, when the aqueous phase comprises electrolytes (e.g., chloride ions, sodium ions, etc.), microparticles with pores on their surface, i.e., microparticles with a porous surface, are formed; illustrative non-limiting examples of aqueous phases containing electrolytes include physiological saline or 0.9% NaCl aqueous solution. This first emulsion (W/O) can be obtained by conventional methods, for example by means of sonication, under suitable conditions or by using techniques and products which can create porosities according to the methodology used in the present invention. The resulting emulsion (W/O) is then mixed with another aqueous phase, for example a polyvinyl alcohol (PVA) aqueous solution and is emulsified by conventional methods, for example by using a turbo homogenizer to produce the double emulsion (W/O/W) under suitable conditions according to the production protocol for each batch of microparticles. The solvent is subsequently removed by suitable conventional methods, for example when the solvent is DCM simply adding an isopropanol solution and stirring for a suitable time period for the purpose of extracting organic solvent residues. The microparticles produced can be collected by means of centrifugation and washed and can subsequently be resuspended in distilled water and filtered to remove the possible nanoparticles that may have been produced during production.

The composition of the invention can be presented in the form of a suspension, advantageously in the form of an aqueous suspension, or said composition of the invention can alternatively and preferably be presented in the form of a freeze-dried product or dry powder for the prior reconstitution thereof before it is used.

The composition of the invention has proven to be useful as a contrast medium for applications in ocular surgery because the microparticle combination with its specific physicochemical surface, size and particle size distribution characteristics induces dynamic and visual properties defined to provide sufficient staining of intraocular structures, such as the vitreous body, the retina, the inner limiting membrane (ILM), etc.

In fact, different assays conducted by the inventors have shown that the composition of the invention, which is the result of a combination of microparticles with particular characteristics in terms of size (mean particle diameter), size distribution and outer surface, have, *inter alia*, the following advantages: it allows delineating the vitreous and more easily extracting it during vitrectomy; it increases the contrast of the inner limiting membrane of the retina during the surgical procedure, thus facilitating the extraction of the vitreous; it allows detecting the vitreous in the posterior chamber of the eye in the event of a capsular rupture during cataract surgery; and it improves intra-surgical viewing in any situation in which increased contrast in transparent or translucent tissues, for example in vitreous tissue, is required. Therefore, the composition of the invention allows viewing the vitreous and the retina under surgical conditions which allows performing safer and more effective surgeries.

In another aspect, the invention relates to an ophthalmic composition, hereinafter ophthalmic composition of the invention, comprising:
a) an effective amount of a composition of the invention, i.e., a composition comprising a plurality of microparticles of a biodegradable polymer, wherein said microparticles have a mean particle diameter comprised between 10 and 60 µm, and wherein between 10% and 40% of the total of said microparticles have a porous surface; and
b) an ophthalmically acceptable carrier.

The composition of the invention is present in the ophthalmic composition of the invention in an effective amount, i.e., in a amount such that when it is placed in the vitreous body it allows viewing intraocular structures, for example the vitreous, the retina, the ILM, etc.

Said ophthalmically acceptable carrier is a non-toxic product. In a particular embodiment, said ophthalmically acceptable carrier comprises an aqueous medium, for example water, such as sterile water for injection, or it is an aqueousbased carrier comprising mostly, i.e., more than 50% (w/v), water.

Additionally, if desired the ophthalmically acceptable carrier comprises at least one additional component in a suitable amount to enhance ocular compatibility or ophthalmic acceptability of the ophthalmic composition in relation to that same ophthalmic composition but without that additional component. Therefore by way of illustration, in a particular embodiment said ophthalmically acceptable carrier comprises an aqueous medium, for example water, and, at least one additional component selected from a buffer, a tonicity agent, a resuspension agent, etc., and mixtures thereof.

A presentation form very useful for the ophthalmic composition of the invention consists of an aqueous suspension comprising, in addition to the composition of the invention, an ophthalmically acceptable liquid aqueous carrier.

In a particular embodiment, said ophthalmically acceptable carrier further comprises a resuspension agent in an effective amount to maintain the suspension for a longer time period or to enhance the stability of the suspension in relation to the same composition but in the absence of said component. Illustrative non-limiting examples of said resuspension agents include cellulose or cellulose derivatives, surfactants, wetting agents, etc.

In another particular embodiment, said ophthalmically acceptable carrier further comprises a buffer for controlling the pH of the composition and/or a tonicity agent in a suitable amount for controlling the osmolality of the composition. The composition preferably comprises a buffer and a tonicity agent. Illustrative non-limiting examples of buffers that can be used include acetate, citrate, phosphate, borate buffers and the like, as well as mixtures thereof. The amount of buffer must be enough to maintain the pH of the composition between approximately 6 and approximately 8, preferably between approximately 7 and approximately 7.5. Illustrative non-limiting examples of tonicity agents include salts, for example sodium chloride, potassium chloride and the like, as well as other ophthalmically acceptable tonicity agents, such as glycerol, mannitol and the like. The amount of tonicity agent must be enough to provide an ophthalmically acceptable osmolality to the composition, for example between 200 and 400, preferably between 250 and 350 mOsmol/kg; advantageously, the ophthalmic composition of the invention is isotonic.

The ophthalmic composition of the invention is advantageously sterile; said composition can be sterilized by conventional methods known by persons skilled in the art, before, during or after packaging or packing.

Said ophthalmic composition of the invention advantageously lacks preservatives and dyes or therapeutic agents exerting pharmacological activity in the eye when the ophthalmic composition is administered.

The ophthalmic composition of the invention can be obtained by conventional methods based on the mixture of its components.

If desired, the ophthalmic composition of the invention can be presented in the form of a kit comprising all or part of the components of the composition. Therefore, in another aspect the invention provides a kit, hereinafter kit of the invention, comprising the composition of the invention. In a particular embodiment, said composition of the invention is present in the form of a freeze-dried product or solid powder and the kit comprises, in addition to the composition of the invention, means for the reconstitution thereof and for forming the ophthalmic composition of the invention. In another particular embodiment, said composition of the invention is present in the form of an aqueous suspension. The kit of the invention can further contain other additional components as mentioned above in relation to the ophthalmic composition of the invention and/or instructions for use.

In another aspect, the invention also relates to the use of said composition of the invention or said ophthalmic composition of the invention. Said use generally comprises placing, for example by means of intravitreal injection, an effective amount of said composition into the vitreous body of a mammal's eye, such as a human being. The ophthalmic composition of the invention acts as a contrast medium in said vitreous body and allows viewing intraocular structures, for example the vitreous, the retina, the ILM, etc., the surgeon can therefore perform the surgical procedure in the eye, for example removing at least part of the vitreous humor of the eye, safely and efficiently because the entire vitreous body as well as the retina and other intraocular structures can be viewed. The administration of the ophthalmic composition of the invention is preferably performed by means of injection, for example by means of intravitreal injection, with a 27G (gauge) or 30G needle.

Therefore, in another aspect the invention relates to the use of the composition of the invention or of the ophthalmic composition of the invention as a contrast medium for viewing intraocular structures in an animal's eye. In a particular embodiment said animal is a mammal, preferably a human being.

In another aspect, the invention relates to a method for viewing the vitreous body of an eye which comprises placing an effective amount of a composition of the invention or of an ophthalmic composition of the invention in the vitreous body of an eye. Expressing this inventive aspect in a different way, the invention relates to a composition of the invention or an ophthalmic composition of the invention for use in viewing the vitreous body of an eye. In a particular embodiment, said eye is an animal's eye, preferably a mammal, more preferably a human being.

In another aspect, the invention relates to a method for staining the vitreous body of an eye which comprises placing an effective amount of a composition of the invention or of an ophthalmic composition of the invention in the vitreous body of an eye. Expressing this inventive aspect in a different way, the invention relates to a composition of the invention or an ophthalmic composition of the invention for the use thereof in staining the vitreous body of an eye. In a particular embodiment, said eye is an animal's eye, preferably a mammal, more preferably a human being.

In another aspect, the invention relates to a method for removing at least part of the vitreous of an eye which comprises placing an effective amount of a composition of the invention or of an ophthalmic composition of the invention in the vitreous body of an eye and removing at least part of the vitreous of the eye. Expressing this inventive aspect in a different way, the invention relates to a composition of the invention or an ophthalmic composition of the invention for the use thereof in removing at least part of the vitreous of an eye. In a particular embodiment, said eye is an animal's eye, preferably a mammal, more preferably a human being. In a particular embodiment, all or substantially all of the vitreous is removed.

This surgical procedure to remove the vitreous of an eye is generally known as a vitrectomy and is recommended in a number of situations, for example *inter alia*, in:
- ocular complications due to diabetes, such as bleeding, retinal detachment, diabetic retinopathy, proliferative diabetic retinopathy;
- vitreous opacity due to different reasons, such as bleeding, inflammatory residues, infections, etc.;
- retinal detachment with rigidity and scar tissue;
- retinal detachment due to vitreoretinal proliferation;
- severe ocular trauma, for example foreign body that has entered into or is piercing the eye;
- macular disorders, for example macular hole, pre-macular membranes, macular cysts, etc.;
- post cataract surgery vitreous changes causing macular edema;
- epiretinal membrane;
- branch vein occlusion;
- posterior uveitis; etc.

Cataract surgery is the most frequently performed ocular surgery worldwide today. The ophthalmic composition of the invention can be suitable for the diagnosis and handling of a capsular rupture, which is one of the most frequent and serious complications that can take place during cataract surgery. Therefore, the ophthalmic composition of the invention can also be used in cataract surgery, particularly in those cases in which complications such as posterior capsular rupture can take place.

The ophthalmic composition of the invention can generally be used for any ocular surgery in which transparent or translucent tissue is involved, for example in vitrectomy and cataract surgery.

The following examples illustrate the invention and must not be considered as limiting thereof.

### EXAMPLE 1

### Production of PLGA microparticles

Several batches of PLGA microparticles were produced by means of the double emulsion-solvent evaporation/extraction method previously described by Igartua [Igartua, M., et al., Gamma-irradiation effects on biopharmaceutical properties of PLGA microspheres loaded with SPf66 synthetic vaccine. Eur J Pharm Biopharm, 2008. 69(2):519-26], with minor modifications. Briefly, a solution of PLGA in dichloromethane (DCM) (50 mg/mL) is emulsified with the aqueous phase [physiological solution (or 0.9% sodium chloride aqueous solution) or sterile water for injection were used according to the batch] by means of sonication at 50 W (Bandelin®, HD-3200) for a time period comprised between 30 and 60 seconds. The resulting emulsion (W/O) was added to 25 mL of 2% polyvinyl alcohol (PVA) aqueous solution and emulsified using a turbo homogenizer (Ultra-turrax® T-25, IKA-Labortechnik) to produce the double emulsion (W/O/W) for a time period comprised between 2 and 5 minutes according to the production protocol for each batch. Finally, 100 mL of 2% isopropanol solution were added and the resulting mixture was stirred according to each batch for a time period comprised between 2 and 4 hours for the purpose of extracting the organic solvents (DCM). The microparticles were then collected by centrifugation (10,000 rpm, 5 minutes) and were washed three times with distilled water for removing the residual PVA. The microparticles were subsequently resuspended in distilled water and filtered for the purpose of removing the possible nanoparticles that may have been produced. The filtration was performed by passing the suspension through a filter with a mesh opening of 8 or 16 µm, depending on the batch. Once the filtration is performed, the two obtained fractions, eluted fraction (which contained the nanoparticles) and the retained fraction (which contained the microparticles and corresponded to the fraction with the best performance), were frozen at -80°C, separately freeze-dried for 49.5 hours, and if necessary, some samples were aliquoted and vacuum-sealed for subsequent evaluation.

Throughout the entire microparticle production process it is advisable to control the morphometric and physicochemical characteristics of the microparticles by means of optical microscopy. The quality control of the product (microparticles) throughout the process was carried out by means of optical microscopy for the purpose of generally and qualitatively identifying the physicochemical characteristics of the microparticles of each production batch before and after filtering. To that end, 5 µL aliquots of the microparticle suspension at different points of the process were stained with 5 µL of Harris hematoxylin stain. The shape and diameter of the microparticles were characterized by means of a Leica DM 2000 compound optical microscope.

Different batches of PLGA microparticles were obtained by varying the aqueous phase used in the production of the first emulsion (W/O) [physiological solution or sterile water for injection] as well as the sonication time, the operating conditions for the production of the double emulsion [speed and duration of the homogenization in the homogenizer], removal of solvents (DCM) or time of contact of the emulsion with isopropanol and the mesh opening of the filter used during filtration. Table 1 shows the operating conditions used in the production of some of the 40 different batches of PLGA microparticles obtained according to the process described above.

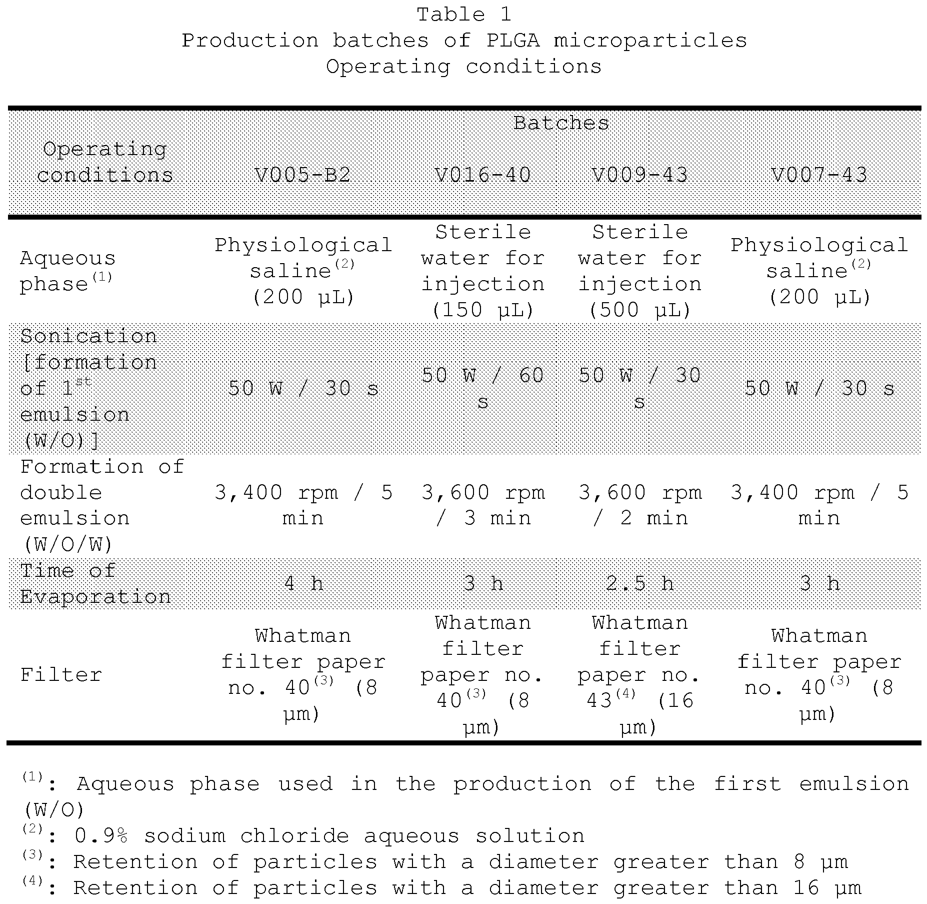

The different batches of PLGA microparticles produced were generally obtained with good production yields (greater than 65%) before filtration. However, after filtration the retained fraction (corresponding to the fraction which contained the microparticles) has high yields (greater than 85%) except in batch V005-B2, which had the smallest yield in the retained fraction and corresponded to the batch of porous microparticles (Table 2).

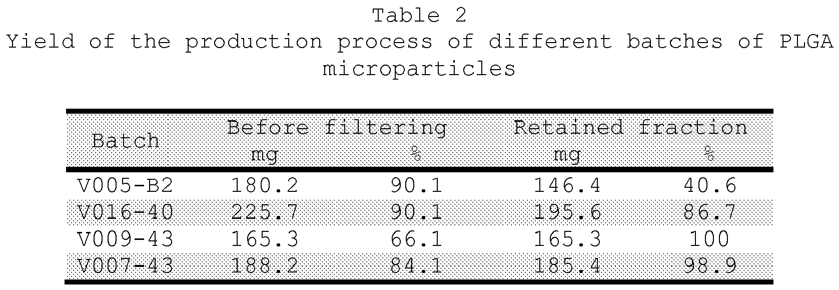

Sometimes the individual or mixed batches of PLGA microparticles (see below) were packaged in glass vials (vials, 25 mg/vial) and subsequently vacuum-sealed with an aluminum capsule and subjected to sterilization by means of gamma radiation with a Co-60 source (Aragogamma, Barcelona, Spain). A dose of 25 kGy was used according to the recommendation of the European Pharmacopoeia for sterilization [European Pharmacopoeia, in 6th, Council of Europe: Strasbourg Cedex, France, 40 2007]. Once the sterilization process ended, sterility is evaluated by means of microbiological control. Once this control is complied with, the product is labeled, being considered as a finished product and is prepared for subsequent *in vitro* and *in vivo* evaluation.

### EXAMPLE 2

### Characterization of PLGA microparticles

The PLGA microparticles obtained according to Example 1 were initially characterized by means of:
a) optical microscopy, and
b) *in vitro* microparticle precipitation and distribution assays

for the purpose of selecting the microparticles which presented suitable performance with respect to viewing, dynamics in the vitreous and handling with surgical instruments. The microparticles selected were subsequently analyzed more thoroughly by means of laser diffraction, scanning electron microscopy (SEM), and Zeta potential. The *in vitro* biodegradation of said microparticles was also analyzed.

### 2.1 Determination of the proportion and sizes of the obtained microparticles by optical microscopy

For the purpose of knowing the physicochemical and morphometric characteristics of the microparticles of each production batch in a first assessment, they were observed by means of optical microscopy. To that end, a 5 µL aliquot of the suspension of PLGA microparticles to be analyzed was stained with 5 µL of Harris hematoxylin stain. The shape and diameter of the microparticles were characterized by means of a Leica DM 2000 compound optical microscope. The results of the mean microparticle diameter of some batches are shown in Table 3 (below).

### 2.2 In vitro microparticle distribution and precipitation assay

Since the microparticles must comply with particular characteristics of adherence to the vitreous humor and characteristics of distribution dynamics through the vitreous humor for use as a contrast medium in vitrectomy, a first selection of the PLGA microparticles obtained in the different batches was performed based on their dynamic and adherence characteristics. To that end, the following microparticle distribution and precipitation assay was performed which seeks to reflect the conditions observed by an ophthalmologist when a microparticle suspension is injected during a vitrectomy.

Briefly, 25 mg of the PLGA microparticles to be analyzed were suspended in 1 mL of physiological solution and the resulting suspension was injected in a vessel with a dark bottom which contained water [500 mL], the effect produced being observed and those microparticles which were mostly deposited on the bottom of the vessel and left a substantially clear and transparent solution being selected for subsequent characterization and study; the microparticles which were not deposited (which sedimented or precipitated) on the bottom but floated in the water forming a type of "cloudiness" were discarded because they would not allow suitable viewing of the vitreous humor or of the nearby tissues (e.g., retina) immediately after injection.

Additionally, the parameter "t₅₀" was defined as the precipitation time of 50% of the microparticles resuspended in the vial for being injected in the vessel containing water. This parameter was determined visually. To calculate the t₅₀, the researcher prepared control suspensions of the microparticles to be assayed, left them to stand for a particular time period and established the time necessary for the particles to precipitate; they were generally left to stand for at least 8 hours for the purpose of assuring that all the microparticles present in the aqueous medium were deposited on the bottom of the vessel, and the approximate height of 100% of the injected microparticles could thereby be calculated; subsequently, once the microparticle suspension to be assayed was calibrated, the researcher then stirred and/or the vial of the microparticle suspension and recorded the time it took for 50% of the microparticles to precipitate in comparison with the corresponding control, those microparticles having t₅₀ equal to or less than 4 minutes being selected.

This assay was initially performed with PLGA microparticles from different batches, and mixtures of PLGA microparticles from different batches were subsequently assayed because it was observed that some microparticles had good adherence properties and others had good dynamic distribution properties, as described below.

The results obtained by means of this *in vitro* microparticle distribution and precipitation assay showed that some microparticles had good precipitation characteristics, whereas other microparticles had good distribution dynamic characteristics. Therefore, the subsequent, more in-depth characterization of said microparticles allowed determining that the microparticles which had pores on their surface [batch V005-B2 (see section 2.4 below)] had good adherence properties and that the microparticles with a mean particle diameter comprised between approximately 10 µm and approximately 60 µm had good distribution dynamic properties, the microparticles with a relatively small mean particle diameter comprised between approximately 10 µm and approximately 20 µm being the microparticles which had good dynamic properties, whereas the microparticles with a relatively large mean particle diameter comprised between approximately 40 µm and approximately 60 µm being the microparticles which had good precipitation properties.

Therefore, since some microparticles had good adherence or precipitation characteristics and other microparticles had good distribution dynamic characteristics, mixtures of PLGA microparticles from different batches were designed for the purpose of developing mixtures of PLGA microparticles with good properties for use as a contrast medium in vitrectomy; to that end, the different batches of microparticles to be included in each of the mixtures were selected based on the properties they provided. Therefore, based on these observations and on the particle size distribution results of the individual batches (see section 2.3) and on *ex vivo* activity assays (Example 4), different mixtures of the batches of microparticles were designed depending on their physicochemical and morphometric characteristics and on their *in vitro* and ex *vivo* results for the purpose of finding a multiparametric product with respect to particle sizes and particle surfaces which offered optimal results as a contrast medium in translucent tissues (e.g., the vitreous). To produce said mixtures, the different batches to be included in each of the mixtures to be produced were selected, and the amounts of PLGA microparticles suitable for each formulation with the idea of producing several units in total of a 25 mg/mL dose for each of the mixtures were weighed; the resulting mixtures were resuspended with sterile water for injection (1000 µL) and were distributed in vials at a proportion of 250 µL in each vial. Said mixtures were analyzed as indicated above to determine the t₅₀ thereof. Table 3 shows the results of some of the selected mixtures as well as the mean particle diameter of each batch determined by optical microscopy.

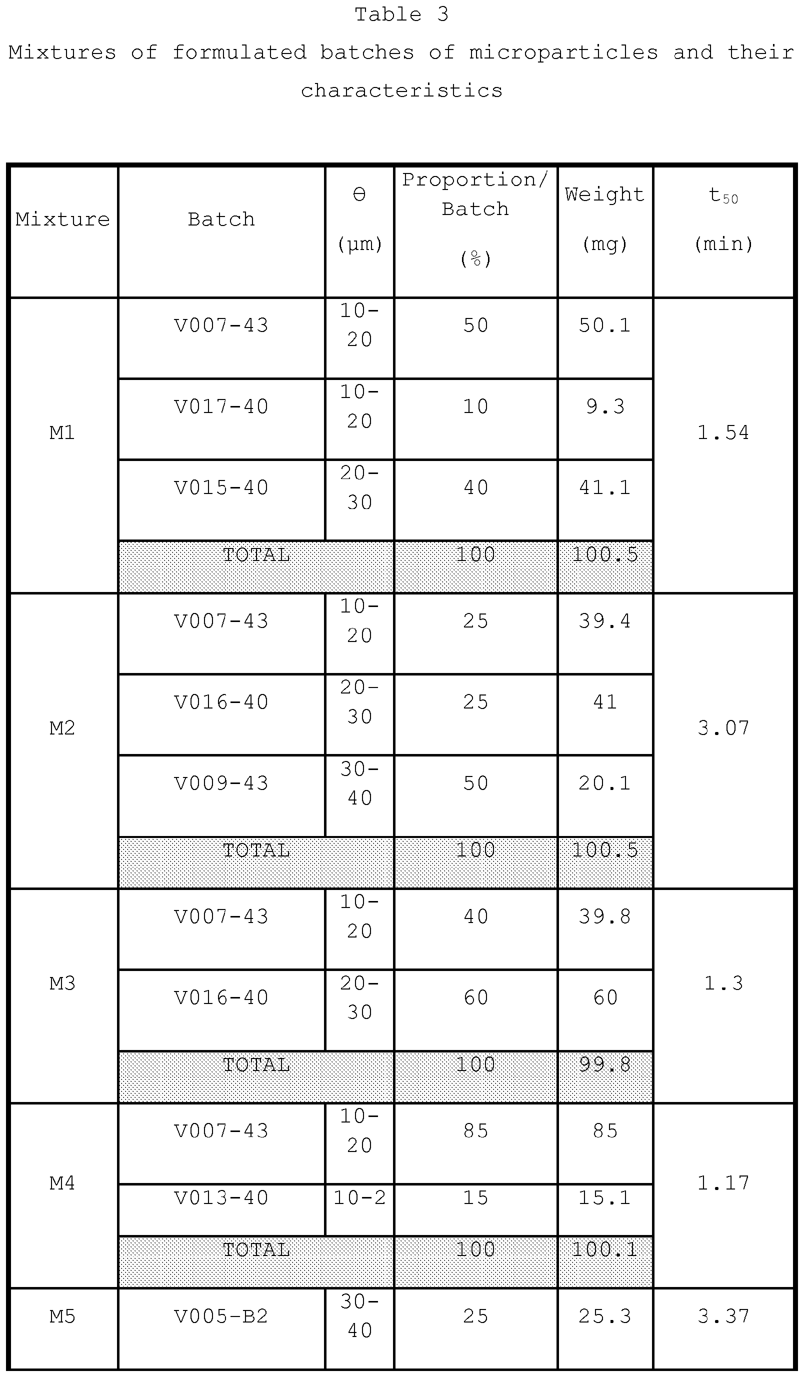

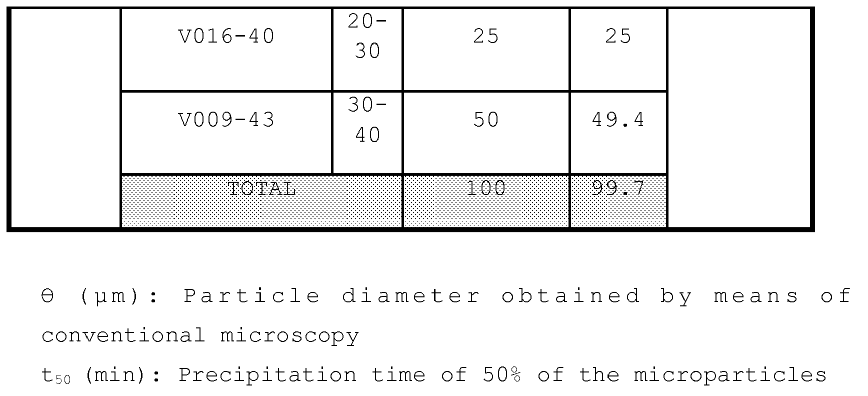

The different batches of microparticles were selected based on the size of most of their microparticle population previously established by means of optical microscopy (diameter of most of the population, θ) [see section 2.1] and surface (presence/absence of pores on the surface) [see section 2.4]. Percentages of each of the batches were subsequently established based on the microparticle sizes and characteristics which are considered important for the development of a composition of microparticles as a contrast medium for use in vitrectomy. The obtained results complemented with the *in vitro* and *ex vivo* studies [Example 4] showed that the mixture identified as M5 was the one that provided the best results for its intravitreal application as a contrast medium for staining the vitreous.

### 2.3 Evaluation and characterization of the mean microparticle diameter distribution by means of laser diffraction

One of the critical factors in obtaining batches of microparticles is their ample particle size (mean diameter) distribution because it induces significant variations with respect to the active ingredient load (where appropriate), the release profile thereof, bioavailability and efficacy. The microparticle size (mean diameter) and microparticle size distribution are important parameters in the characterization of the microparticulate systems and, in this case, they are a fundamental part due to the fact that depending on those parameters, the final formulation can be optimized with respect to proportions and sizes of microparticles to be included in the final product according to the results obtained in the *in vitro* microparticle distribution and precipitation assay.

According to the prior particle size distribution results of different individual batches, *in vitro* microparticle precipitation and distribution assays and their *ex vivo* activity [Example 4], different mixtures of the batches of microparticles were designed depending on their physicochemical and morphometric characteristics, as mentioned above. As mentioned above, Mixture 5 (the composition of which is shown in Table 3) was the one which presented optimal conditions for intravitreal application as a contrast medium.

Therefore, said Mixture 5 and its components were more thoroughly characterized. To that end, the particle size distribution of Mixture 5 (M5) and of each of the individual batches forming it in terms of size and distribution of the microparticle subpopulations in the final mixture were analyzed as described below.

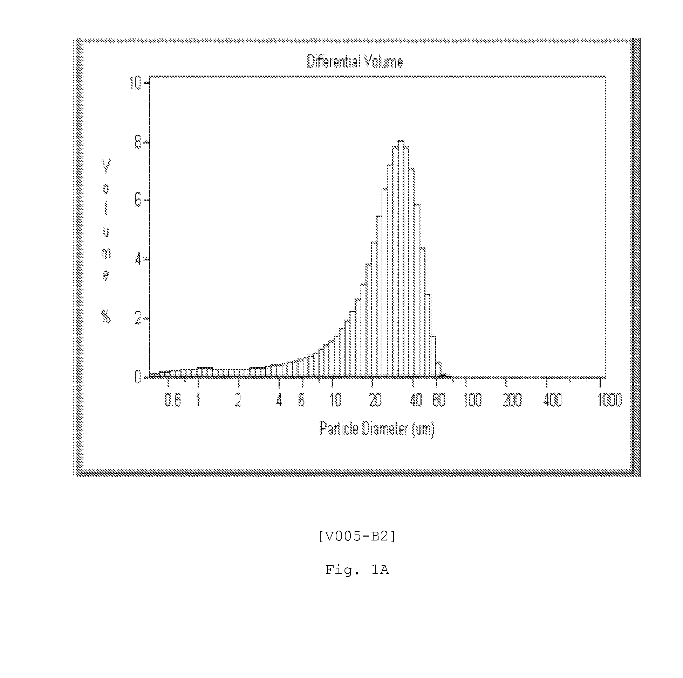

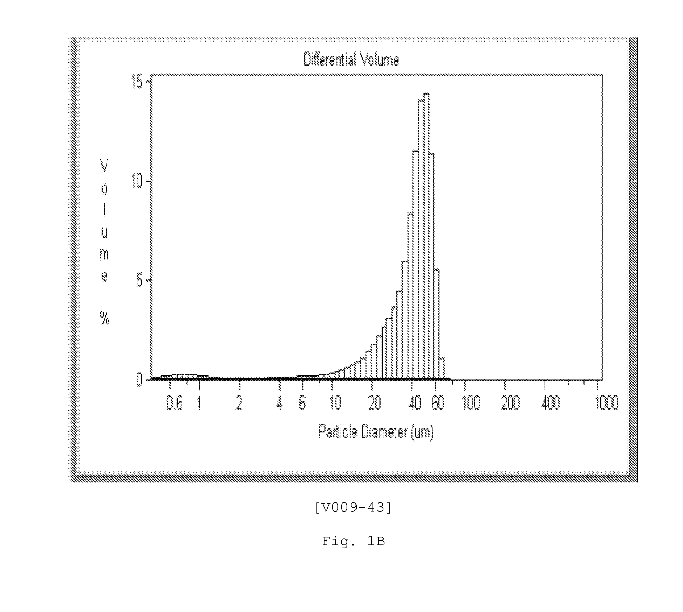

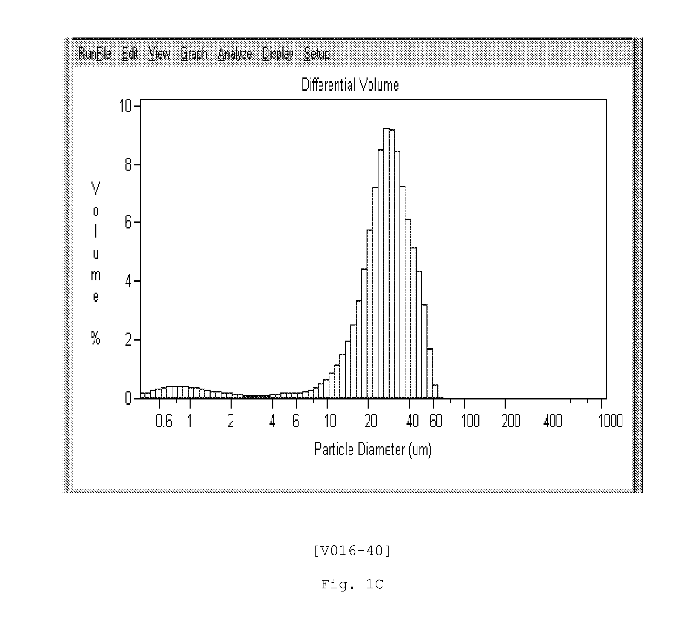

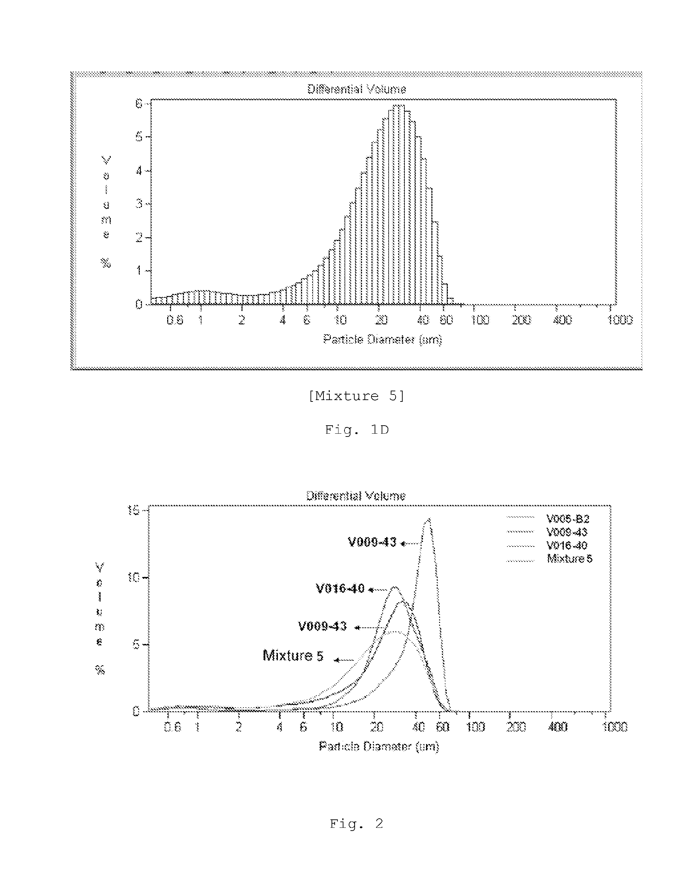

The particle size (mean diameter) distribution was determined by means of laser diffraction using a Coulter Counter ® LS130 particle analyzer (Amhers, MA, USA) which uses a laser radiation source at 750 nm wavelength and measures particles in the range comprised between 0.4 and 900 µm in diameter by means of the Fraunhofer approximation. The samples are dispersed in 0.1% PVA and measured in water dispersing the samples with stirring and performing the measurement without stirring. Laser diffraction allows establishing a descriptive histogram of the characteristics at the level of microparticle size and population volume corresponding to each size expressed percentage-wise. Figure 1 shows the mean microparticle diameter distribution for each of the three batches forming Mixture 5 [V005-B2 (Figure 1A), V016-40 (Figure 1B) and V009-43 (Figure 1C) at a proportion of 25:25:50, respectively) and finally the overlay thereof with respect to the particle size distribution profile previously obtained for the mixture [Mixture 5 (Figure 1D)].

The results shown in Figure 2 were obtained upon comparison by means of overlaying the particle size distribution profiles obtained by laser diffraction analysis of the individual batches forming part of Mixture 5 and the particle size distribution profile of Mixture 5.

Laser diffraction demonstrated the difference between the microparticle samples with respect to particle size and distribution of the different particle subpopulations in one and the same batch. Therefore and according to the histograms, it can be seen that the particle size of batches V005-B2 and V016-40 describe a similar particle size profile with slight differences with respect to the distribution of particle subpopulations in each of the batches. It should be mentioned that in relation to batch V005-B2, the main difference of this batch with respect to the other two batches is with respect to the surface of the microparticle because in its production protocol [Table 1] small modifications were made to generate microparticles with surface porosities according to what is designed to include in the mixture; these differences were verified by means of specialized analytical techniques (SEM) as described below [see section 2.4]. Batch V009-43 is the one that provides the most significant differences to the mixture with respect to the particle size because this batch has a large particle size (19-57 µm) compared with the other two batches (7-44 µm) described above.

Upon comparison of the particle size distribution profile of Mixture 5, it is demonstrated how each of the three individual batches contributes by inducing synergism in the different particle size subpopulations generating a moderately asymmetrical particle size distribution profile with a left side bias (positive bias) and covering a broad particle size range with very similar volume percentages. Most of the population has particle diameters of 20-40 µm. The mixture describes a wide particle size distribution profile.

The results obtained with the different laser diffraction determinations are summarized in Table 4.

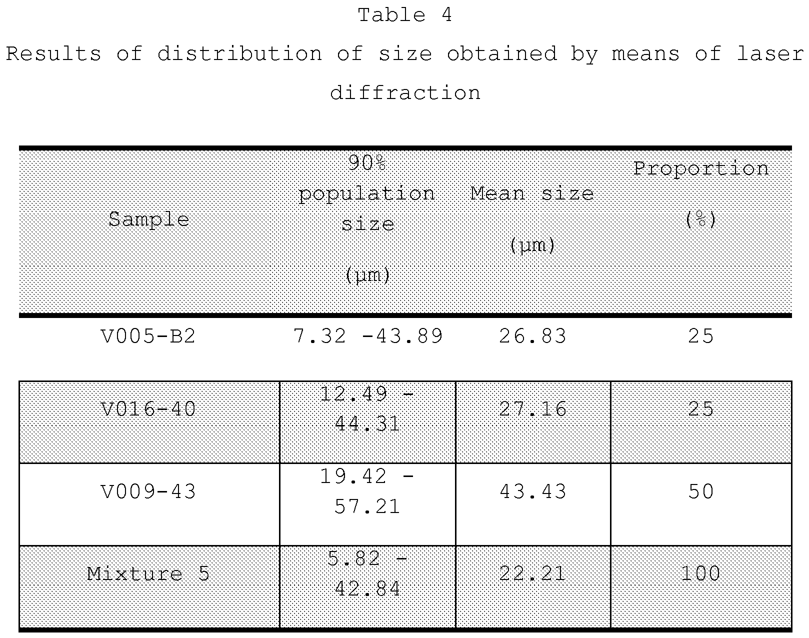

In summary, it is observed that each of the batches and Mixture 5 have a different mean particle diameter distribution within a range comprised between 5 and 44 µm, showing averages of particle size comprised between 22 and 43 µm. According to the particle size distribution results obtained with the different samples and according to the *ex vivo* assays (in enucleated pig eyes) [Example 4], it could be concluded that the good characteristics of Mixture 5 as a contrast medium were provided based on the particular characteristics of each batch. Therefore batch V005-B2 provides characteristics with regard to porosity and hydrophilic characteristics; batch V009-43 provides large particle size populations; and batch V016-40 contributed to increasing the microparticle subpopulations, mainly of those with diameters between 20 and 30 µm. Considering all these characteristics, the final product obtained as a contrast medium results from the mixture of three batches of microparticles with particular characteristics contributed by each to the final mixture with respect to shape, size and surface within a broad range of microparticle size between 13-42 µm, wherein there are both microparticles with a smooth surface and microparticles with pores on the surface.

### 2.4 Evaluation and characterization of the microparticles by means of SEM

Mixture 5 demonstrated in *in vitro* and ex *vivo* studies that it is a good candidate as a contrast medium during vitrectomy, so its characterization with respect to morphology, surface and mean particle diameter, *inter alia*, generates interest from the point of view of functionality of the product as a contrast medium. Therefore the different batches of microparticles forming Mixture 5 as well as Mixture 5 itself were characterized by means of scanning electron microscopy (SEM). The methodology of analysis includes fixing the samples on double-sided carbon tape which is located on an aluminum support, coating it with gold in argon atmosphere, and subsequently analyzing the samples using a scanning electron microscope (Hitachi S3400).

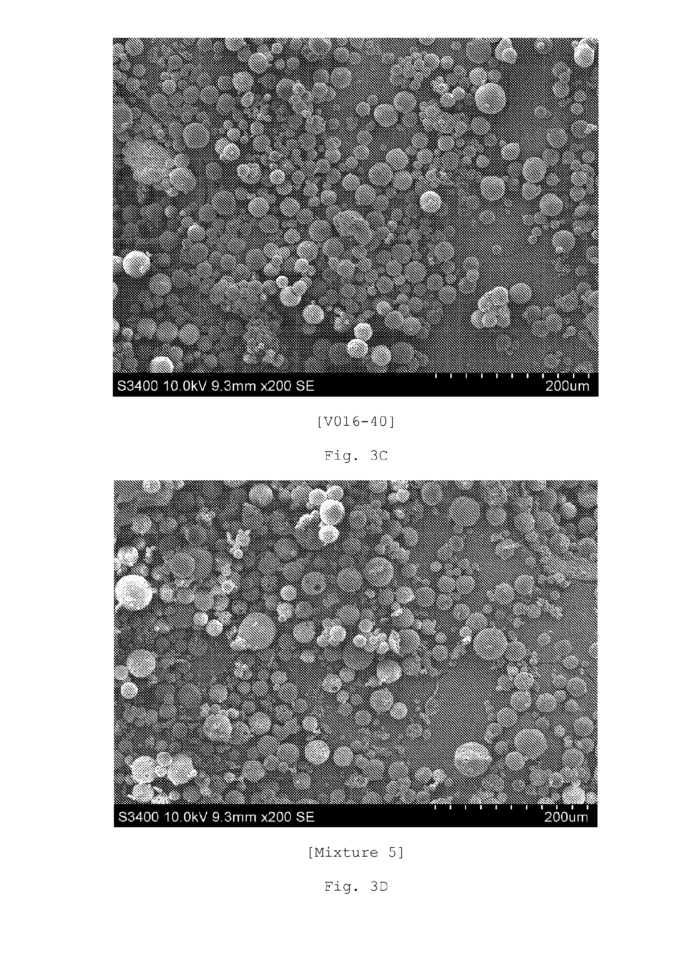

Figure 3 shows how batch V005-B2 has porous microparticles with a heterogeneous microparticle population having sizes less than 50 µm. Batch V009-43 is the batch with the most homogeneous particle size distribution of the three batches forming Mixture 5 and it has the largest microparticles (>50 µm) of the three batches included in Mixture 5. Batch V016-40 has microparticle sizes that are also less than 50 µm like batch V005-B2 does, but unlike the latter, it has smooth surfaces. Finally, the microphotograph of Mixture 5 shows microparticles with considerable dispersion in terms of particle size as a result of the mixture of the three batches described above and therefore resulting in a mixture of their morphological characteristics relating to size and surface.

Upon comparison of the results obtained by means of the SEM technique with the particle size distribution results previously obtained by means of laser diffraction, it is demonstrated how batches V005-B2 and V016-40 have greater particle size dispersion, as is seen in the microphotographs. It was also determined how the microparticles of batch V009-43 are the microparticles with the smallest size dispersion and correspond to mostly large microparticles. Finally, Mixture 5 has microparticle size dispersion and surface characteristics corresponding to the heterogeneity which was induced by means of combining the particular characteristics of each batch of microparticles.

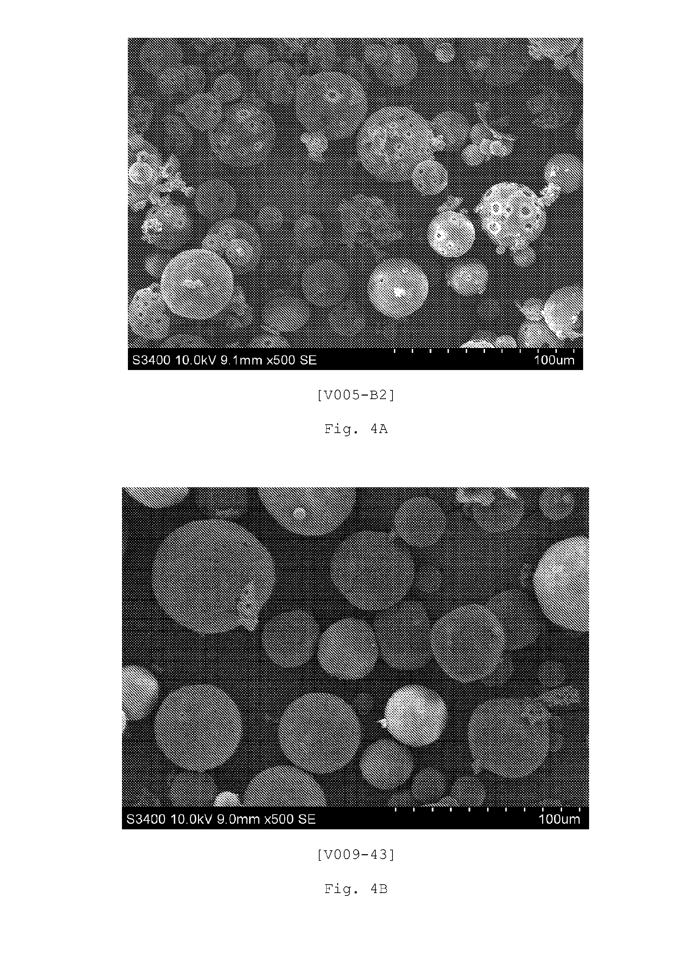

Figure 4 describes more the surface characteristics and in some cases the inside of the microparticles in greater detail. The microphotographs of batch V005-B2 (Figure 4A) allow determining that it is formed by porous microparticles at the surface level with a heterogeneous and random distribution of pores; it is also shown that they are internally porous microparticles. A variety of pores are distinguished with regard to surface pores, some microparticles have small pores, other microparticles have large and deep pores, whereas other microparticles have a variety of porosities in one same microparticle. Batch V009-43 clearly shows its superiority in size with respect to the other batches forming Mixture 5; it is obvious with the microphotograph of said batch (Figure 4B) that some of the microparticles of the samples during the analysis by means of SEM presented deformation, an effect due to the fact that the scanning electron microscopy (SEM) electron beam modified the shape of the samples, suggesting that they are soft microparticles that are sensitive to the electron beam of the technique, however it should be pointed out that all the samples analyzed by means of SEM have a spherical shape under normal conditions. The microphotograph of that sample (V009-43) allowed determining that the inside of those microparticles is porous. In summary, batch V009-43 is homogenous with respect to size, it being the batch having the largest particles with respect to the other batches included in Mixture 5. Batch V016-40 also has microparticles with smooth surfaces with size dispersion (Figure 4C) but, unlike batch V009-43, it has size distribution and microparticle populations similar to batch V005-B2. Finally, the sample corresponding to Mixture 5 shows microparticle heterogeneity with respect to size and surface which ended up being interesting characteristics as a contrast medium.

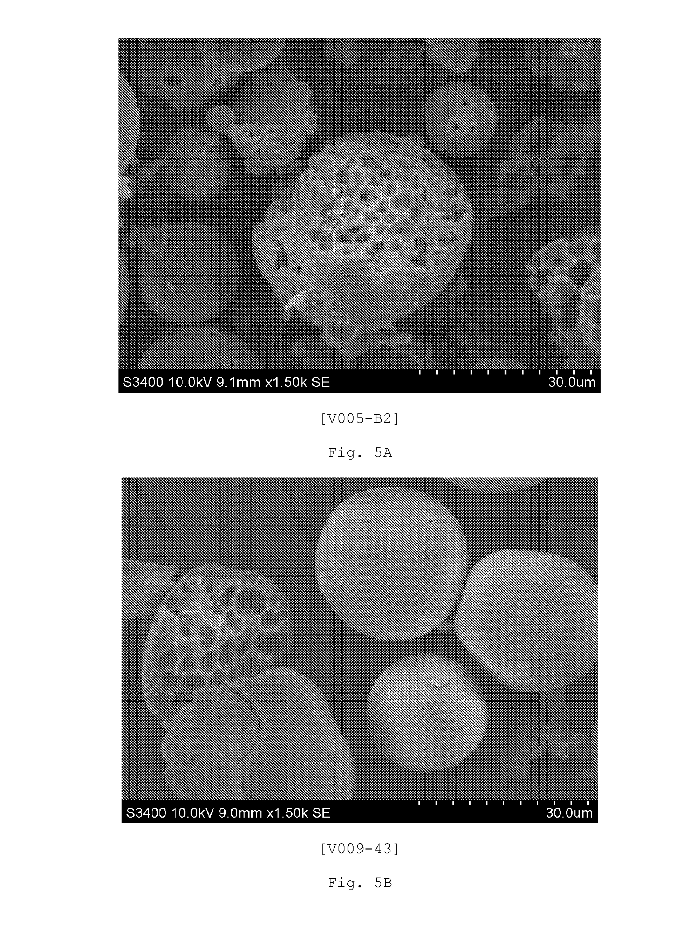

With respect to the inside of these microparticles, it was determined that they have a highly porous internal structure as a result of the microphotographs where in some cases fractioned microparticles can be identified, exposing the interior thereof (Figure 5). According to the obtained results it could be established that the diversity of sizes within a range of 10-60 µm, together with the combination of smooth and porous surfaces of the microparticles, conferred to Mixture 5 particular properties as a contrast medium in transparent tissues such as the vitreous. The porosity of batch V005-B2 plays an essential role in comparison with the other batches included in Mixture 5. 2.5 Characterization of the microparticles with respect to the Zeta potential

The Zeta potential is one of the essential characteristics of the microparticles under study due to the fact that as a result of it, the performance and interaction of the microparticles in suspension can be predicted. The Zeta potential is the measurement of the charge a short distance from the surface, wherein the molecules of the dispersant (water, buffer, etc.) move with respect to the molecules of the microparticle surface boundary and will interact according to the potential in this layer. The magnitude of the Zeta potential gives an idea as to the stability of a suspension. All the particles in suspension have a highly positive or highly negative Zeta potential, whereby the particles tend to repel one another, preventing the occurrence of flocculation or the formation of aggregates. If in contrast the particles have low Zeta potential values without inducing sufficient repulsive force between the particles, flocculation is present. It has generally been established that a "stabile" suspension has a Zeta potential value greater than 30 mV or less than -30 mV in a pH less than 4 or greater than 7.5 according to the literature [Malvern. *Zeta Potential theory.* Undated [cited 20110324]; available at http://www.malvern.com].

Due to the fact that the Zeta potential is a readily measurable characteristic [Sjöström, B., et al., Structures of nanoparticles prepared from oil-in-water emulsions. Pharm Res, 1995. 12 (1) :39-48; Washington, C., Photon correlation spectroscopy. Particle Size Analysis in Pharmaceutics and Other Industries. 1992, New York: Ellis Horwood], it has been used to draw conclusions as to the physical stability or instability of formulations under different conditions, for example the effect of its storage, or under interaction with electrolytes, or simulations with biological fluids [Freitas, C. & R.H. Muller, Effect of light and temperature on zeta potential and physical stability in solid lipid nanoparticle (SLN) dispersions. Int J Pharm, 1998. 168:221 - 229; Freitas, C. & R.H. Muller, Stability determination of solid lipid nanoparticles (SLN) in aqueous dispersion after addition of electrolyte. J Microencapsul, 1999. 16(1):59-71; Jenning, V. et al., Vitamin A-loaded solid lipid nanoparticles for topical use: drug release properties. J Control Release, 2000. 66(2-3):115-26; Zimmermann, E. & R.H. Muller, Electrolyte- and pH-stabilities of aqueous solid lipid nanoparticle (SLN) dispersions in artificial gastrointestinal media. Eur J Pharm Biopharm, 2001. 52(2):203-10]. Studies of the effect of different preparations and their influence on the Zeta potential have also been conducted and translated in terms of stability and/or flocculation, and it is also used to determine the effect that processes such as freeze-drying or sterilization can cause on the Zeta potential [Cavalli, R., et al., Sterilization and freeze-drying of drug-free and drug-loaded solid lipid nanoparticles. Int J Pharm. Int J Pharm, 1997. 148:47-54; Lim, S.J. & C.K. Kim, Formulation parameters determining the physicochemical characteristics of solid lipid nanoparticles loaded with all-trans retinoic acid. Int J Pharm, 2002. 243(1-2):135-46; Pinto, J.F. & R.H. Muller, Pellets as carriers of solid lipid nanoparticles (SLN) for oral administration of drugs. Pharmazie, 1999. 54:506-509; Schwarz, C. & W. Mehnert, Freeze-drying of drug-free and drug-loaded solid lipid nanoparticles (SLN) . Int J Pharm, 1997. 157 (2) :171-179; Trotta, M., F. Debernardi, and O. Caputo, Preparation of solid lipid nanoparticles by a solvent emulsification-diffusion technique. Int J Pharm, 2003. 257(1-2):153-60; Venkateswarlu, V. & K. Manjunath, Preparation, characterization and in vitro release kinetics of clozapine solid lipid nanoparticles. J Control Release, 2004. 95(3):627-38]. Other studies have been conducted to optimize the microparticle elimination time or to reduce the phagocytosis by manipulating the Zeta potential or the microparticle surface. Particularly, the determination of the Zeta potential at the pharmaceutical level is indicative of performances with respect to determination of stability of a formulation, studies of effects induced during the freeze-drying or sterilization process, particle-macrophage binding studies, particle aggregation studies and effects on different modified release dosage forms.

The Zeta potential is determined by means of laser Doppler anemometry (measurement of the force or speed) using a Malvern® Zetasizer Nano ZS Model ZEN 3600 (MALVERN INSTRUMENTS Ltd.) provided with a photon correlation spectrophotometer. The Zeta potential of the microparticle suspension of Mixture 5 was determined in triplicate. The samples of the microparticles were resuspended in sterile water for injection, the value obtained being the mean of 3 determinations. According to the results obtained by means of laser Doppler anemometry it was determined that Mixture 5 has a Zeta potential (ζ) of -27.9 mV and a viscosity of 0.08872 cP at 25°C.

The negative Zeta potential value of the PLGA microparticles can be attributed to the carboxyl-terminal groups of the copolymer which are not susceptible to ionization. This result coincides with other reports in the literature in which PLGA microspheres presented a negative surface charge [Jaganathan, K.S. and S.P. Vyas, Strong systemic and mucosal immune responses to surface-modified PLGA microspheres containing recombinant hepatitis B antigen administered intranasally. Vaccine, 2006. 24(19):4201-11].

In addition, the contact or addition of electrolytes induces a significant electrolyte concentration-dependent effect on the Zeta potential.

Among the physical properties of a tissue contrast system by means of injection, a stable suspension to prevent the formation of aggregates and easy injection are required. Nevertheless, these characteristics must be accompanied by others which allow deposition on the tissues. In the case of Mixture 5, a product with mean dispersion characteristics was sought because a highly dispersed product would present very slow sedimentation, which would not favor delineating the retina within an optimal time in the operating room. Since the vitreous is mainly formed of collagen, hyaluronic acid, water and electrolytes, it is to be expected that when Mixture 5 comes into contact with the vitreous, a change in the Zeta potential of the mixture is generated. Mixture 5 will thereby be modified, inducing an instability effect on the product because its original Zeta potential value is at the limits where it is considered a stable suspension (-27 mV), enhancing the sedimentation effect of the product on the retina. This process of instability due to Zeta potential changes with respect to the formation of aggregates is an interesting characteristic in Mixture 5 from the point of view of functionality, and in vitrectomy it is important that it does not occur prior to the injection in order to thus prevent occlusion in the syringe.

With respect to viscosity values, it should be pointed out that Mixture 5 is more fluid than water because water has a higher viscosity (1.0020 cP at 20°C) than Mixture 5 does (0.08872 cP at 25°C); since Mixture 5 is more fluid than water, its dispersion and distribution through the vitreous body which is formed by 99% water would be facilitated. Since the Zeta potential value of Mixture 5 is negative, it would be advisable to resuspend said Mixture 5 in sterile water for injection and thereby not induce modifications in the Zeta potential which alter the physicochemical properties thereof.

### 2.6 In vitro determination of the biodegradation of the contrast medium over time

*In vitro* assays were conducted for the purpose of establishing the time it would take for the samples of PLGA microparticles to degrade. Briefly, 10 mg of the samples to be studied (individual batches of microparticles or mixtures thereof) were weighed in 7 new, sterile, 1.5 mL Eppendorf tubes (each tube corresponds to a week of degradation samples), 500 µL of a 20 mm phosphate buffer solution (PBS), pH 7.4, were added to each tube and Eppendorf tube number 7 was incubated for 49 days at 37°C under continuous orbital stirring , tube number 6 for 42 days and so on and so forth until tube number 1. Once the incubation time of each tube (tube one corresponding to week one and so on and so forth until tube seven for week seven) has been completed, each sample was frozen at -80°C, freeze-dried and stored until completing collection of all the samples for a total of seven samples and the degradation of the residual microparticles was subsequently determined at microscopic level by means of SEM.

Upon examining the SEM in terms of morphology of the particles at the start time (zero days), it was observed that the product is dispersed as individual, well-defined sphericalshaped microparticles with a size distribution between 5 and 60 µm. The following samples corresponding to the following weeks of degradation show a reduction in the microparticle population, demonstrating that the degradation is directly proportional to the passage of time, a reduction in proportion and in size being evident, and acquiring a slight loss of shape, becoming oval and adhesive or sticky. All this occurs within the first 4 weeks of degradation because for the last evaluated weeks, the microparticles form a small, amorphous-shaped aggregate that is difficult to disperse. These samples, particularly batch V005-B2, have hygroscopic characteristics making them very cohesive at the intra-particle level. The degradation kinetics of batch V005-B2 used to perform microscopic follow-up of the degradation of the product by means of SEM is illustrated below.

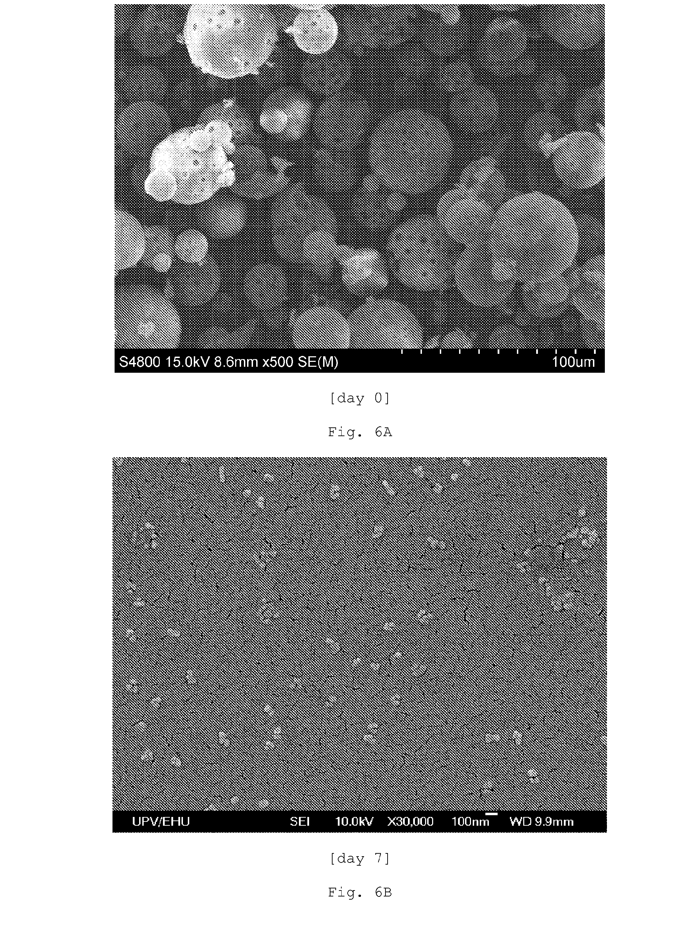

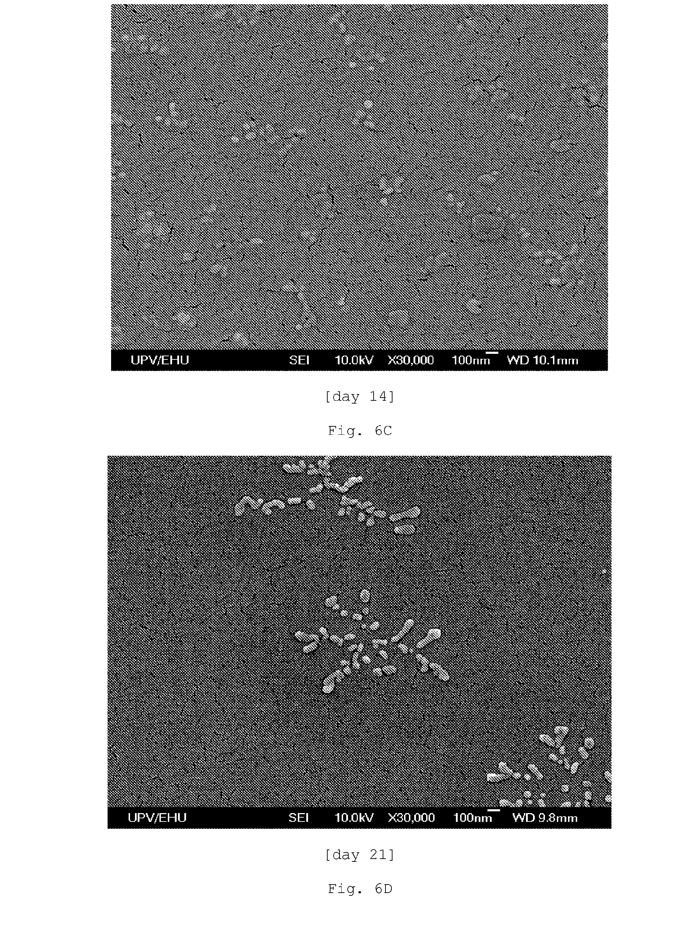

Figure 6 illustrates the time-dependent microparticle size reduction of batch V005-B2. The presence of microparticles up until day 21 becomes evident because from day 28 it could not be detected by means of SEM because no microparticles were observed and their degradation products have hygroscopic properties, acquiring a hard-to-handle sticky texture, indicating that the sample has completely degraded. These analyses allow concluding that the microparticles of this batch V005-B2 take between 21 and 28 days to degrade. The different microparticle samples prepared with 50:50 PLGA polymer generally have degradation times comprised between 5 and 6 weeks, the microparticles as such disappearing.

### EXAMPLE 3

### In vitro characterization of PLGA microparticles by means of cell culture of retinal cell lines

To evaluate the possible toxicity for the use of the PLGA microparticles [Example 1] at the cell level an ARPE-19 retinal pigment epithelial cell culture was exposed to different concentrations and its proliferation capacity was evaluated. The studied dilutions of Mixture 5 were 1:1000, 1:500, 1:100, 1:50 and 1:10.

ARPE-19 cell line: Cells of the ARPE-19 (Human Retinal Pigment Epithelial) cell line [Dunn, K.C., et al., ARPE-19, a human retinal pigment epithelial cell line with differentiated properties. Exp Eye Res, 1996. 62(2):155-69], acquired in the ATCC (American Type Culture Collection - Manassas, Virginia, USA) were used to conduct *in vitro* toxicological studies. The cells were seeded on a 24-well plate at a concentration of 60,000 cells/well leaving them in an initial incubation process of 12 hours so that the cells adhere to the plate and being maintained for synchronization for 16 hours before treating them with each of the evaluated microparticle dilutions. The cells were cultured in standard conditions (atmospheric humidity of 5% CO₂ at 37°C) in a culture media mixture at a ratio of 1:1 (DMEM (Dulbecco's Modified Eagle's Medium) and Ham's F-12) supplemented with 10% bovine serum, 2 mM L-glutamine and 1% streptomycin/penicillin. The cells were detached by means of a solution resulting from the combination of 0.5% trypsin and 0.2% ethylenediaminetetraacetic acid (EDTA), and they were subsequently sub-cultured twice a week in a 1:3-1:4 dilution.

Cell treatment with the microparticles: Vials containing 25 mg of microparticles were resuspended with 1 mL of phosphate buffer saline (PBS) (Suspension Stock of 25 mg/mL), from which dilutions were made with the culture medium of the ARPE-19 cells (DMEM + Ham's F-12 at a ratio of 1:1). The evaluated dilutions were:
1/10→ 2.5 mg/mL
1/50→ 0.5 mg/mL
1/100→ 0.25 mg/mL
1/500→ 0.05 mg/mL
1/1000→ 0.025 mg/mL

Determination of cell viability: The effect induced by the PLGA microparticles on the ARPE-19 cell line was analyzed by means of the MTT kit (tetrazolium bromide of Sigma-Aldrich). MTT is reduced by cytosolic and mitochondrial dehydrogenases of the living cells forming the formazan complex (purple colored) which is stoichiometrically representative of cell viability. Three hours after incubating the cell line with MTT, the MTT solution is removed and 100 µL of dimethylsulfoxide (DMSO) are added to each well. The optical density is measured by means of a microplate reader (ELx800 Microplate Reader, BioTek® Instruments, Winooski, USA) at 540 nm. The results were statistically analyzed by means of the t-test and the significance was p ≤ 0.05.

Effect on cell viability due to the effect of a single exposure of the cell line to PLGA microparticles: The effect of five different dilutions of PLGA microparticles on the ARPE-19 cells was evaluated. The follow-up was performed for 10 days without adding microparticles in each subculture made. The results of said assay showed that when ARPE-19 pigment epithelial cells are cultured in the presence of different dilutions of the preparation of PLGA microparticles after 3 days of treatment, there is a statistically significant delay in cell proliferation (especially for the 1:10 or 2.5 mg/mL dilution).

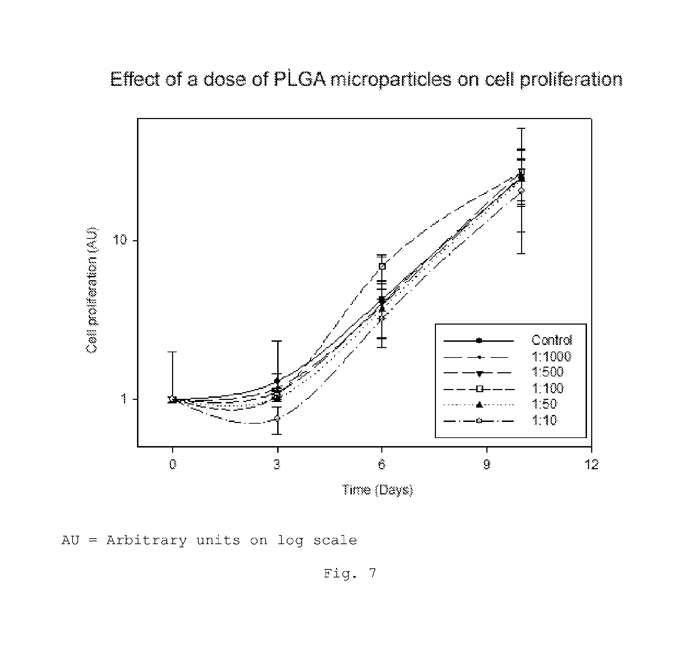

However, as subcultures are made, the effect of the microparticles on cell proliferation is reduced, and after 10 days of incubation with all the evaluated dilutions no statistically significant effects on cell proliferation are observed, which points towards an absence of toxicity at the cellular level due to the prolonged exposure to the product (Mixture 5). This is possibly due to the effect of the biodegradable characteristics of the polymer (Figure 7). The degradation of the product is therefore evident and no significant differences were established between any of the dilutions used and the control culture (or culture in the absence of the preparation).

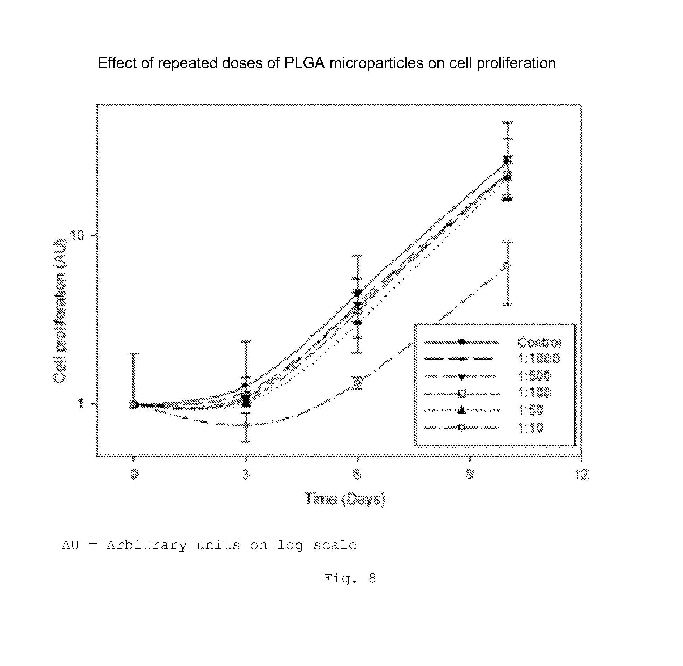

Effect on cell viability due to the effect of repeated exposures of the cell line to PLGA microparticles: In a second assay ARPE-19 cells were cultured in the presence of different dilutions of the PLGA preparation but this time adding the same amount of the preparation each time a pass was made on the cell culture (3, 6 and 10 days). The results demonstrated that in addition to the statistically significant delay in cell proliferation which was described in the first assay taking place after 3 days of treatment, a delay in the proliferation became evident after 6 days of treatment for the 1:50 (or 0.5 mg/mL) dilution and after 6 and 10 days for the 1:10 (or 2.5 mg/mL) dilution. The other dilutions reach values similar to the control, which would indicate that high doses could induce a reduction in cell proliferation (Figure 8).

The results obtained with the two assays could indicate that an injection of Mixture 5 would not affect cell proliferation over time with any of the dilutions used, because there is degradation thereof in the intraocular cavity. However, it would not be advisable to perform serial injections to prevent the accumulation of the PLGA microparticles that may induce an effect on the proliferation of the ARPE-19 cell line. It should additionally be pointed out that during the normal practice of vitrectomy the contrast medium (e.g., Mixture 5) would be extracted together with the vitreous during surgery, which further reduces the possibility of the product inducing a toxic effect due to the fact that high proportions of the product do not remain in the posterior segment.

### EXAMPLE 4

### Ex vivo evaluation and characterization in enucleated pig eyes to determine the contrast effect during vitrectomy

*Ex vivo* assays were conducted in enucleated pig eyes in the operating room to evaluate the contrast effect of Mixture 5 during vitrectomy. To that end, briefly, the surgical procedure of vitrectomy was performed in enucleated pig eyes using as a vitreous contrast medium the intravitreal injection of each of the batches of PLGA microparticles or Mixture 5 using a dose of 25 mg/mL and evaluating its intravitreous performance as a contrast medium. The evaluated parameters were:
- Dynamics: The dynamics of the substance to be injected conditions the dynamic performance thereof at the time of the injection, i.e., how it is distributed once it has been injected in the vitreous cavity (depending on how this distribution is, some tissues or others will be marked).
- Viewing: The substance must be easily detectable after injection because its function is to adhere to transparent or barely visible tissues in their natural state, in order to thus increase the contrast thereof and facilitate their handling during vitrectomy.
- Surgical material: different types of substances can be injected today into the vitreous during vitrectomy; as the substance to be used is more easily handled, its application during the surgical procedure will be simpler.

*Ex vivo* assays were conducted for the purpose of verifying the functionality of the different microparticles and Mixture 5 as a contrast medium, using Triamcinolone (TA) [the commercial product used today as a contrast medium during vitrectomies] as a positive control.

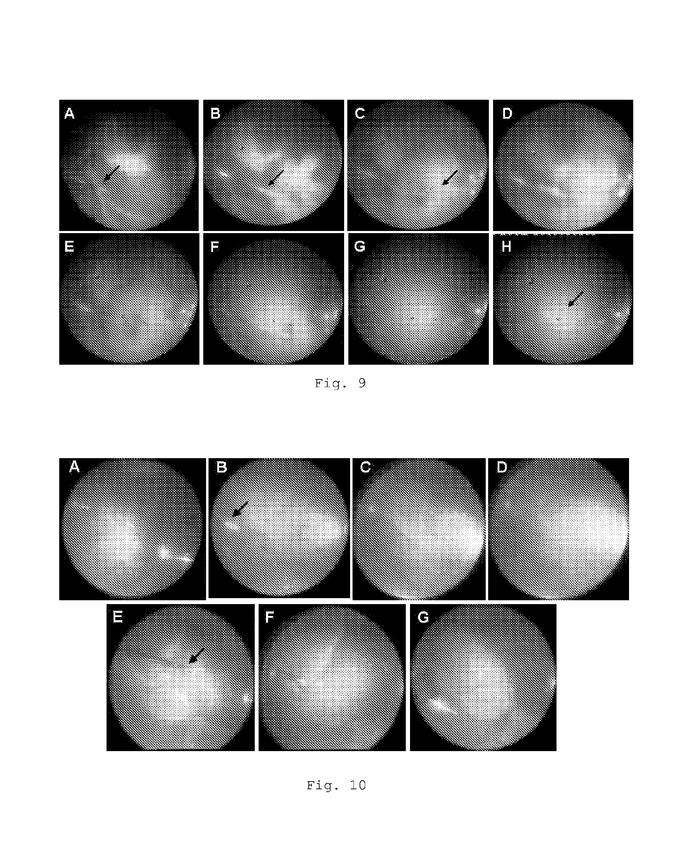

Figure 9 chronologically illustrates the application of TA during a vitrectomy in enucleated pig eyes. Microphotograph 9A shows the blood vessels of the posterior segment as well as part of the retina before the injection (the arrow indicates the TA injection needle). Figure 9B shows the start time of a second TA injection. Microphotograph 9C indicates the dispersion halos which are formed in the vitreous upon the injection of the TA and microphotograph 9D illustrates how the crystals of the TA are decanted towards the retina in the form of a product cloud. Microphotographs 9E and 9F indicate the dynamics and the distribution of the product in the posterior segment being distributed on the retina. Microphotograph 9H reflects how the TA has colored the vitreous generating a white cloud, and in microphotograph 9G the arrow indicates how a second injection of the product also describes the retinal sedimentation performance.

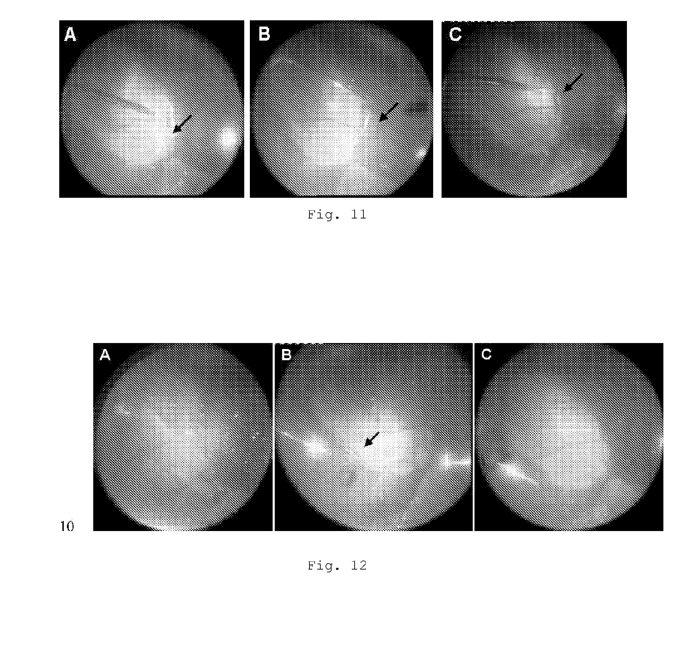

Figure 10 chronologically illustrates the application of Mixture 5 of microparticles during a vitrectomy in enucleated pig eyes. Microphotograph 10A shows the blood vessels of the posterior segment as well as part of the retina. Figure 10B shows the needle with which the microparticle-based product is injected. Microphotographs 10C and 10D reflect the dynamics and distribution of the product in the posterior segment being distributed through the vitreous humor. Figures 10E-10F illustrate the extraction of the vitreous body simultaneously with the previously injected microparticles. The arrow in microphotograph 10E indicates the vitrectome extracting the vitreous. The largest microparticles are deposited on the retina, delineating the epiretinal membranes (see below, Figure 11), helping the surgeon to view them and therefore assuring the patient's safety.

Figure 11 illustrates Mixture 5 deposited on the epiretinal membranes which have not previously been extracted. Said precipitate is mainly formed by particles having sizes greater than 30 µm according to preliminary assays. The arrows reflect the decrease of the amount of product extracted sequentially. A second injection of Mixture 5 can additionally be performed to verify and optimize the complete extraction of the vitreous.

Figure 12 demonstrates the efficacy of the product for detecting small residues of the vitreous humor still present in the anterior segment. Microphotograph 12A shows the distribution of the product and the slight stain caused due to the fact that it is an eye in which a central vitrectomy has already been performed. A less dense distribution of the particles is shown, and for the most part they tend to precipitate because they cannot find any vitreous in which they can be resuspended. However, the microparticles finding a slight presence of microfibrils of the vitreous humor mass have an effective association with the vitreal fibers, making them visible, "stain." the vitreous white (microphotograph 12B, arrow) making it evident for removal. Figure 12C shows the almost complete extraction of the product (Mixture 5) which allows clearly viewing the blood vessels and the retina.

Table 5 summarizes some of the different *in vitro* and *ex vivo* assays conducted, detailing the results obtained with the different evaluated mixtures.

**Table 5 Results of the mixtures of batches of microparticles evaluated in vitro and ex vivo**

| Mixture | θ | In dark container | | Enucleated eyes | | | |
|---|---|---|---|---|---|---|---|
| | | t₅₀ | Obs. | Syringe and needle | Viewing | Dynamics | Comments |
| M1 | 8-37 | 1'54" | (1) | Loaded with 25G cannula | Vitreous ok | Easily diffused | (5) |
| M2 | 11-47 | 3'07" | (2) | Injected with 25G cannula without problems | Initially the entire field clouds over, delineating the entire vitreous | Very dense and without precipitating | (6) |
| M3 | 11-39 | 1'30" | (3) | Occlusion required use of several insulin cannulas | The entire vitreous is cloudy and fewer instruments are used | Diffused throughout entire vitreous | (7) |
| M4 | 12-49 | 1'17" | (3) | Occlusion required use of short silicone cannula | OK | OK | (8) |
| M5 | 5-43 | 3'27" | (3) | Injected with short silicone cannula | OK | OK | (9) |
| TA | | | (4) | Loaded with 25G cannula | OK | Little diffusion, very dense sample | (10) |

| | | | | | | | |
|---|---|---|---|---|---|---|---|
| θ: Mean particle diameter (µm) t₅₀: Precipitation time of approximately 50% of the population TA: Triamcinolone *: Does not precipitate OK: Good (1): Easy sample dispersion and loading (2): Not possible to load with 25G; Loaded with pink 30G cannula (3): Loaded with pink 30G cannula (4): Loaded with 25G cannula (5): No precipitation of the cannula and it precipitates, the particles being observed clearly upon moving the light closer (6): Easily diffused but does not precipitate, very little observed on the fundus (7): Precipitates well and diffuses throughout the entire vitreous; had a better profile than M2 (8): Required two needle changes before changing to the short silicon cannula (9): The best mixture with excellent distribution and marking of the vitreous and hyaloids (10): Very dense stable cloud in the posterior hyaloids; precipitates very well; most precipitates and diffuses very little; very dense | | | | | | | |

### EXAMPLE 5

### In vivo evaluation and characterization in rabbits

The safety, efficacy and functionality of PLGA microparticles were evaluated *in vivo.* The results of the preliminary assay for the *in vivo* characterization of the effect induced by the microparticles was performed in rabbits as an animal model as described below.

Animals: The animals remained in the animal house receiving food and care according to the guidelines on using animals for research from the association for Research in Vision and Opthalmology" (ARVO) and research animal protection laws in force in Spain (RD 1201/2005). The safety, efficacy and functionality of the PLGA microparticles was evaluated *in vivo.*

The rabbits were anesthetized with an intramuscular injection of a mixture of ketamine hydrochloride (37.5 mg/kg; Ketolar® Parke-Davis S.A., Barcelona, Spain) and with xylazine hydrochloride (5 mg/kg; Rompun®, Bayer AG, Leverkusen, Germany). 0.5% tetracaine hydrochloride and 1 mg of oxybuprocaine (Colircusi Anestésico Doble, Alcon-Cusi, S.A., Barcelona, Spain) were used as a topical anesthesia. 16 pigmented rabbits distributed as indicated in Table 6 were used.

**Table 6**

| Groups and distribution of animals of the *in vivo* assay | | |
|---|---|---|
| Groups | Right eye | Left eye |
| Group 1 (11 rabbits) | Injection of microparticles | Control |
| Group 2 (3 rabbits) | Injection of BSS | Control |
| Group 3 (2 rabbits) | Control | Control |

| | | |
|---|---|---|
| Microparticles: The microparticles used corresponded to batch V007-43 3 (Table 1) BSS: balanced saline solution | | |

Suspension: 25 mg/ml doses of microparticles were evaluated *in vivo*. The freeze-dried product of PLGA microparticles was reconstituted with physiological solution and mixed thoroughly using a vortex until obtaining a suspension. A dose of 40 µl/eye was injected in each animal. Pigmented rabbits were used, which facilitates clinical follow-up of the microparticles in the vitreous, and they were dilated with a drop of tropicamide to perform better tracking of the injected solution. The pupils were dilated before the injections and prior to all the examinations of the fundus of the eye with a drop of tropicamide.

Intravitreal injection: The intravitreal injection was performed at 5-6 mm but instead of injecting towards the papilla, it was injected towards the posterior segment to prevent damaging the lens. One of the animals was not subjected to intravitreal injection as it was the control animal.

Clinical follow-up: The clinical follow-up was performed by analyzing different parameters:
- the conjunctiva, anterior chamber, lens and anterior vitreous were analyzed under the light of a surgical microscope;
- the fundus of the eye was analyzed under indirect ophthalmoscopy with a 20-dioptre lens;
- intraocular pressure was subjectively measured by means of digital tonometry, also analyzing other non-specific signs such as corneal transparency.

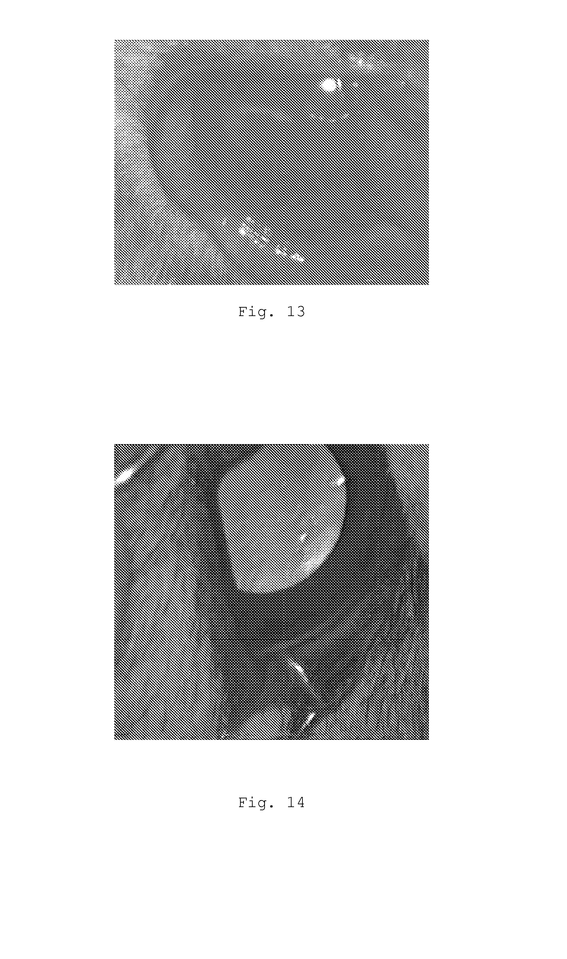

It was therefore possible to establish a mild conjunctival reaction in all the eyes during the first week post-injection (Figure 13). No evidence of reaction to the injected product was observed in the anterior chamber under inspection with the surgical microscope. Traumatic cataracts were not found in any eye. In the vitreous, only small whitish particles were viewed in the area of the incision the first days after the injection both in the eyes of the rabbits where the microparticles were injected and in the eyes where balanced saline solution (BSS) was injected.

In terms of viewing the retina under indirect ophthalmoscopy, no sign of inflammation or bleeding and no signs of ischemia (Figure 14) were observed. Without modifying any parameter with respect to the basal situation, no ocular hypertensions or corneal disorders were found in the control animals or in the animals corresponding to the evaluation group for the product (V007-43).

### EXAMPLE 6

### Tissue staining in animal model (rabbits)

Histological studies were performed to determine:
- possible effects at the cellular level (viability and/or apoptosis);
- possible effects at the immunological level (inflammation); and/or
- presence of contrast medium in the different structures of the eye.

To that end, once the month after treatment by means of intravitreal injection in the animals (rabbits) had concluded, the animals were sacrificed with intracardial injection of sodium pentobarbital, the eyeballs were enucleated and fixed in 10% buffered formaldehyde for 48 hours and subsequently washed in 0.1 M phosphate buffer for another 24 hours at 4°C. The samples were then processed by performing dehydration with alcohols at increasing concentrations, and a first inclusion in paraffin using a tissue processor (Leica TP 1020, Cat. No. 0704 37101, Leica Microsystems, Nussloch, Germany).

The final inclusion was performed in a paraffin dispenser (Oxford Trade, Casa Álvarez, Madrid), obtaining paraffin blocks at 60°C (ref. C1616VO-95, Duerolab, Salamanca, Spain). The eyeballs were cut in half in order for the paraffin to better infiltrate them.

The microtomy was carried out with a Minot type microtome and serial sections of 5 µm thick which were stained with hemotoxylin-eosin (H-E), were obtained. The preparations were examined with an Olympus B-H optical microscope (Olympus Optical Co. LTD., Tokyo, Japan), and the following parameters were evaluated:
- histological disorders in the conjunctiva; and
- cell death.

The histological observations showed a slight infiltration in some areas of the conjunctiva and in some sections, most likely corresponding to the injection area, whereas no disorders or differences between the control and the treated animals were observed in the cornea or sclera in the fibrous layer.

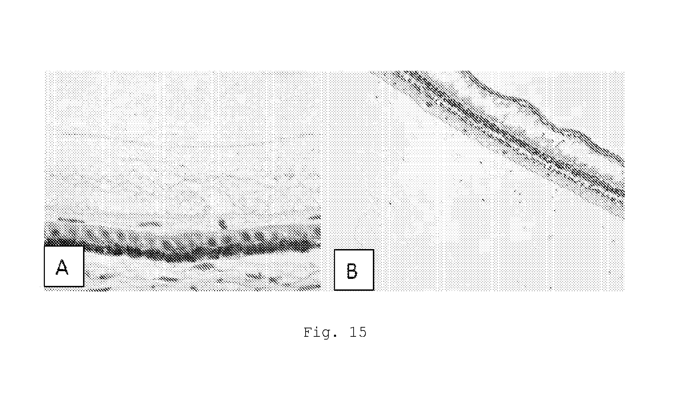

In one section small areas of cell destruction in the choroid were observed, possibly corresponding to injection areas. Some slightly edematous ciliary processes were observed in control and treated animals. Residues of the injected microparticles were observed in the vitreous that were similar to a fine black powder that is adhered close to the pars plana and in the most peripheral part of the vitreous humor. Cell debris were found in the peripheral part of the vitreous. Both observations are similar in both the animals injected with BSS and in the animals injected with microspheres (Figure 15A). No inflammatory reactions or retinal deposits were observed (Figure 15B).

According to the results of the *in vivo* assays and of the histological study it can be concluded that the assayed product (V007-43), representative of the present invention, is an innocuous, compatible, biodegradable and safe product in intravitreal application as a result of the evidence that no alterations of any of the parameters evaluated in the *in vivo* study in comparison with the control injection are observed.

## Claims

1. A composition comprising a plurality of microparticles of a biodegradable polymer, wherein said microparticles have a mean particle diameter comprised between 10 and 60 µm, and wherein between 10% and 40% of the total of said microparticles have a porous surface.

2. The composition according to claim 1, wherein said microparticles have a mean particle diameter comprised between 15 and 50 µm, preferably between 20 and 40 µm.

3. The composition according to claim 1, wherein said biodegradable polymer is selected from the group consisting of polylactic acid (PLA), polyglycolic acid (PGA), lactic acid-glycolic acid or poly-(lactic-co-glycolic) acid (PLGA) copolymer, polyhydroxybutyric acid, polyhydroxyvaleric acid, polycaprolactone, hydroxybutyric acid-glycolic acid copolymer, lactic acid-caprolactone copolymer, polyethylene succinate, polybutylene succinate, starch, starch derivatives, cellulose, acetylcellulose, ethylcellulose, hydroxypropyl methylcellulose, chitosan, chitin, dextran and mixtures thereof, preferably PLGA.

4. The composition according to claim 1 in the form of a freeze-dried product or dry powder, or in the form of a suspension, preferably in the form of an aqueous suspension.

5. An ophthalmic composition comprising:
a) an effective amount of a composition according to any of claims 1 to 4; and
b) an ophthalmically acceptable carrier.

6. The composition according to claim 5, wherein said ophthalmically acceptable carrier comprises an aqueous medium.

7. The composition according to claim 5 or 6, wherein said ophthalmically acceptable carrier comprises at least one additional component selected from the group consisting of a buffer, a tonicity agent, a resuspension agent, and mixtures thereof.

8. The composition according to claim 5 in the form of an aqueous suspension.

9. A kit comprising a composition according to any of claims 1 to 3, or according to any of claims 4 to 7.

10. The kit according to claim 9, wherein said composition according to any of claims 1 to 3 is in the form of a freeze-dried product or solid powder and the kit further comprises means for the reconstitution thereof.

11. The kit according to claim 9, wherein said composition according to any of claims 1 to 3 is in the form of an aqueous suspension.

12. A composition according to any of claims 1 to 8 for use as a contrast medium for viewing intraocular structures in an animal's eye.

13. A composition according to any of claims 1 to 8 for use in:
- viewing the vitreous body of an eye; or in
- staining the vitreous body of an eye; or in
- removing at least part of the vitreous of an eye.

14. A composition for use according to claim 13 in ocular surgery wherein transparent or translucent tissue is involved preferably in vitrectomy and cataract surgery.

15. The composition for use according to claim 14, wherein said ocular surgery is due to:
- ocular complications due to diabetes;
- vitreous opacity;
- retinal detachment;
- severe ocular trauma;
- macular disorders;
- post cataract surgery vitreous changes causing macular edema;
- epiretinal membrane;
- branch vein occlusion; or
- posterior uveitis.
